# EUROPÄISCHE PATENTSCHRIFT

(11) **EP 3 986 651 B1**
(45) Veröffentlichungstag und Bekanntmachung des Hinweises auf die Patenterteilung: **03.01.2024**
(21) Anmeldenummer: 20764458.4
(22) Anmeldetag: 07.08.2020
(51) Int. Cl.: B23D 43/02, B23D 67/04, A61B 17/16, B23B 51/00, B23B 45/00

(54) **BOHRADAPTER**
DRILLING ADAPTOR
ADAPTATEUR DE PERÇAGE

(30) Priorität: 18.06.2019 DE 102019208875
(43) Veröffentlichungstag der Anmeldung: 27.04.2022
(73) Patentinhaber: Fraunhofer-Gesellschaft zur Förderung der angewandten Forschung e.V., 80686 München (DE)
(72) Erfinder: SCHWARZ, Oliver, 70569 Stuttgart (DE); SCHIEBL, Jonas, 70569 Stuttgart (DE); MIKLOSOVIC, Miroslav, 70569 Stuttgart (DE); VOGEL, Alexander, 70569 Stuttgart (DE); KAIMING, Cao, 70569 Stuttgart (DE)
(74) Vertreter: Pfenning, Meinig & Partner mbB
(86) Internationale Anmeldenummer: PCT/IB2020/057472
(87) Internationale Veröffentlichungsnummer: WO 2020/255109

(56) Entgegenhaltungen:
- DE-B3-102013 111 200
- US-A- 4 431 062

## Beschreibung

Die Erfindung betrifft einen Bohradapter zum Aufsetzen auf einen Antriebsmechanismus.

US 4 431 062 A offenbart einen Bohradapter nach dem Oberbegriff des Anspruchs 1.

Das Darstellen von eckigen Löchern ist durch klassische Methoden nur schwer möglich. Dabei werden eckige Löcher in einer Reihe von Anwendungen entweder benötigt oder wären zumindest vorteilhaft (Kavitäten für Implantatschäfte, Dübelbohrungen, Steckverbindungen im Möbelbau, Zierelemente), da diese verdrehsichere Verbindungen ermöglichen oder aus kosmetischen Gründen.

Desweiteren existieren Anwendungsfälle, bei denen klassische rotierende Bohrverfahren aufgrund der Drehmomentenwirkung eine Gefahr für den Nutzer darstellen (Erdbohrer) oder aufgrund des nötigen Anpressdrucks ermüdend für den Nutzer sind.

Bei der Metallbearbeitung kommen teilweise Pendelfräsen zum Einsatz, wobei jedoch verrundete Ecken nicht vermieden werden können. Außerdem werden Räumwerkzeuge eingesetzt, wenn eine Durchgängigkeit der Kavität gegeben ist. Zur Bearbeitung von Hölzern werden eckige Löcher dagegen häufig in Handarbeit gefertigt. Alternativ werden in Einzelfällen Stemmbohrer zum Erstellen rechteckiger Löcher verwendet.

Eine weitere Technik, um Andruckkräfte zu reduzieren, Drehmomente zu vermeiden und eckige Löcher herzustellen, ist das sogenannte Pendelhubbohren (siehe DE 10 2013 111 200). Dies ist eine Technik, bei welcher sich Bohrlanzen (ähnlich Räumwerkzeugen) mit einer nahezu beliebigen Querschnittsform (Dreieckig, Viereckig, Sechseckig, Kreisförmig, Oval etc.) alternierend auf und ab bewegen.

In der DE 10 2013 111 200 sind die Bohrlanzen an einem Antriebsmechanismus fest angeordnet. Es ist nicht vorgesehen, die Lanzen zu wechseln. Derartige Vorrichtungen sind also normalerweise auf eine Lanzengeometrie festgelegt, können also nur Löcher mit einer bestimmten Querschnittsfläche erzeugen.

Aufgabe der vorliegenden Erfindung ist es, einen Bohradapter anzugeben, der es erlaubt, Lanzen mit verschiedenen Geometrien zu verwenden, und der dadurch eine höhere Flexibilität ermöglicht.

Die Aufgabe wird gelöst durch den Bohradapter nach Anspruch 1 und das Bohrsystem nach Anspruch 17.

Die jeweiligen abhängigen Ansprüche geben vorteilhafte Weiterbildungen des erfindungsgemäßen Bohreradapters und des erfindungsgemäßen Bohrersystems an.

Die Erfindung betrifft einen Bohradapter zum Aufsetzen auf einen Antriebsmechanismus. Ein solcher Antriebsmechanismus kann grundsätzlich jede Art von Antrieb sein, vorzugsweise ist er jedoch ein solcher Antrieb, der eine Drehbewegung bewirkt.

Der Bohradapter weist erfindungsgemäß ein Verbindungselement auf, das mit dem Antriebsmechanismus verbindbar ist. Das Verbindungselement ist um eine Verbindungselementachse drehbar. Vorteilhaft kann das Verbindungselement eine Welle sein. Eine solche kann auf einfache Weise an einem Antriebsmechanismus angeordnet werden und durch eine Drehbewegung des Antriebsmechanismus zur Drehung um die Verbindungselementachse angetrieben werden, die dann vorzugsweise koaxial liegt zur Längsachse der Welle.

Erfindungsgemäß weist der Bohradapter außerdem zumindest zwei Bohrlanzen auf, die um eine Mittelachse angeordnet sind. Die Bohrlanzen können vorteilhaft länglich ausgestaltet sein. In diesem Fall kann die Mittelachse parallel liegen zu den Längsrichtungen der Bohrlanzen. Besonders bevorzugt stehen die Längsrichtungen der Bohrlanzen auch zueinander parallel. Die Mittelachse soll hier als mathematische Achse, also als Gerade, verstanden werden.

Der erfindungsgemäße Bohradapter weist außerdem einen Linearisierungsmechanismus auf, mit dem eine Rotation des Verbindungselementes in oszillierende Bewegungen der Bohrlanzen in Richtung parallel zur Mittelachse übertragbar ist. Die oszillierende Bewegung ist also eine Bewegung, die abwechselnd in zwei einander entgegengesetzte Richtungen verläuft, wobei diese Richtungen zu der Mittelachse parallel liegen. Vorzugsweise ist die oszillierende Bewegung periodisch, wiederholt sich also nach einer Periodendauer.

Die Bewegung der einzelnen Lanzen wird durch den Linearisierungsmechanismus vorgegeben. Bevorzugterweise bewegen sich die Bohrlanzen nicht synchron in dem Sinne, dass sie zu jedem Zeitpunkt die gleiche Höhe haben, sondern führen Bewegungen aus, die voneinander unabhängig sind und/oder phasenversetzt dem gleichen Bewegungsmuster folgen. Vorteilhaft kann der Phasenversatz zwischen zwei entlang eines Umfangs um die Mittelachse benachbarter Lanzen 2 π geteilt durch die Anzahl der Lanzen betragen.

In allen Ausgestaltungen der Erfindung, in denen Abgriffselemente vorgesehen sind, können sind diese vorteilhaft mit gleichen Winkelabständen zueinander um die Mittelachse angeordnet sein. Der Winkel zwischen zwei benachbarten Abgriffselementen kann dann also vorteilhaft 2 π geteilt durch die Anzahl der Lanzenaufnehmer betragen. Es kann aber auch vorteilhaft sein, wenn die Abgriffselemente mit ungleichmäßigen Winkelabständen um die Mittelachse angeordnet sind. Zwei Winkel zwischen den Abgriffselementen können dabei gleich sein, aber kleiner als der dritte entstehende Winkel. Vorteilhafterweise sind zwei Winkel ähnlich oder gleich groß aber viel kleiner als ein dritter Winkel. Beispielsweise können die Winkel 60 °- 60°-240° betragen.

Die Lanzenaufnehmer können wie beschrieben jeweils mit einer Sinusfunktion in Abhängigkeit vom Winkel um die Mittelachse verschoben werden. Bevorzugt ist jedoch eine Verschiebung, die abschnittsweise einer Sinusfunktion folgt, zwischen sinusförmigen Abschnitten jedoch Plateauphasen aufweist, in der die Lanzen ihre Position in Richtung der Mittelachse nicht verändern, während das Verbindungselement weiter gedreht wird.

Vorteilhaft kann auch eine Ausgestaltung sein, in der die Winkelabstände benachbarter Abgriffselemente unterschiedlich groß sind. Es können also einige der zueinander benachbarten Abgriffselemente geringere Winkelabstände aufweisen als andere zu einander benachbarte Abgriffselemente.

Erfindungsgemäß weist der Bohradapter außerdem einen Schnellspannmechanismus auf, der für jede der Bohrlanzen einen Lanzenaufnehmer aufweist. Die Bohrlanzen sind jeweils an einem der Lanzenaufnehmer lösbar befestigbar. Durch den Schnellspannmechanismus ist es möglich, die Bohrlanzen schnell auszutauschen und durch Bohrlanzen beispielsweise anderer Geometrien zu ersetzen. Als Bohradapter kann hier der Bohradapter mit Verbindungselement, Linearisierungsmechanismus, Schnellspannmechanismus sowie den Bohrlanzen angesehen werden oder aber auch die Kombination von Verbindungselement, Linearisierungsmechanismus und Schnellspannmechanismus ohne Bohrlanzen. Der Bohradapter kann auch als eine Menge enthaltend das Verbindungselement, den Linearisierungsmechanismus, den Schnellspannmechanismus sowie zwei oder mehr Sätze von Bohrlanzen angesehen werden, wobei jeder Satz eine der Anzahl der Lanzenaufnehmer entsprechende Zahl von Bohrlanzen enthält, die gemeinsam eine bestimmte Bohrlochgeometrie bewirken.

In einer vorteilhaften Ausgestaltung der Erfindung kann der Linearisierungsmechanismus ein Führungssystem aufweisen, das mit dem Verbindungselement verbunden ist. Die Verbindung kann dabei so hergestellt sein, dass eine Rotation des Verbindungselementes auf das Führungssystem übertragbar ist. Der Linearisierungsmechanismus kann außerdem für jeden der Lanzenaufnehmer ein mit dem entsprechenden Lanzenaufnehmer gekoppeltes Abgriffselement aufweisen. Dabei können die Abgriffselemente jeweils in eine Richtung parallel zur Mittelachse verschiebbar sein. Insbesondere kann eine Verschiebung der Abgriffselemente zu einer Verschiebung des entsprechenden Lanzenaufnehmers in Richtung parallel zur Mittelachse führen. Die Abgriffselemente können dann mit dem Führungssystem gekoppelt sein oder an dem Führungssystem angreifen, so dass bei Rotation des Verbindungselementes um die Verbindungselementachse die Abgriffselemente in Richtung parallel zur Mittelachse verschoben werden. Durch diesen Aufbau kann der Linearisierungsmechanismus die Rotation des Verbindungselementes in Linearbewegungen der Lanzenaufnehmer in Richtung parallel zur Mittelachse überführen. Sind die Lanzen in den Lanzenaufnehmern eingesetzt, so kann also der Linearisierungsmechanismus auf diese Weise die Rotationsbewegung des Verbindungselementes in Linearbewegungen der Lanzen in Richtung parallel zur Mittelachse überführen.

Vorteilhafterweise kann das besagte Führungssystem des Linearisierungsmechanismus zumindest eine Bahn oder Nut aufweisen, auf oder in welcher die Abgriffselemente um die Mittelachse herum gleiten oder abrollen. Ist das Führungssystem hier eine Bahn, so können die Abgriffselemente auf der Bahn gleiten oder abrollen. Ist das Führungssystem hier eine Nut, so können die Abgriffselemente in dieser Nut gleiten oder abrollen. Die Abgriffselemente können im Falle von Abrollen als Wälzlager bzw. Rollen ausgestaltet sein, und im Falle von Gleiten als Gleiter. Die Abgriffselemente können entweder fest auf der Bahn gleiten (bereits beschrieben) oder aber einen drehbarbaren Teil enthalten (Fall Abrollen). In diesem Fall können Wälzlager (und als Sonderform hiervon Kurvenrollen) oder Gleitlager zum Einsatz kommen.

Sind die Abgriffselemente als Rollen ausgestaltet, so können vorteilhaft Drehachsen dieser Rollen senkrecht zur Mittelachse stehen. Die Bahn oder Nut kann vorteilhaft in Projektion auf eine zur Mittelachse senkrechte Ebene kreisförmig sein, wobei sie jedoch vorteilhafterweise zumindest entlang eines Teils ihres Umlaufes gegenüber dieser Ebene um einen Winkel von ungleich 0 geneigt ist.

Vorteilhafterweise umläuft die Bahn oder die Nut die Mittelachse. Besonders bevorzugt verläuft die Mittelachse durch den Mittelpunkt einer Projektion der Bahn oder der Nut auf eine zur Mittelachse senkrechte Ebene. Die Bahn oder die Nut ist vorzugsweise mit zumindest Teilen ihrer Länge um die Mittelachse geneigt gegenüber einer Ebene, die senkrecht auf der Mittelachse steht. Dabei kann die Bahn selbst teilweise oder vollständig in einer Ebene laufen, welche Ebene nicht senkrecht und nicht parallel zur Mittelachse steht. Je nach gewünschter Bewegung der Lanzenaufnehmer bzw. Lanzen kann die Bahn vorteilhaft auch unterschiedlich geneigte Abschnitte aufweisen, also Abschnitte, die in unterschiedlichen Winkeln zur Mittelachse oder zu einer zur Mittelachse senkrechten Ebene verlaufen. Betrachtet man also die Position der Bahn oder der Nut in Richtung der Mittelachse, also den Verlauf der Höhe gemessen in Richtung der Mittelachse entlang des Umfangs um die Mittelachse, so kann diese unterschiedliche Funktionen beschreiben. Verläuft beispielsweise die Nut oder die Bahn selbst vollständig in einer Ebene, welche nicht parallel und nicht senkrecht zur Mittelachse steht, so ist diese Funktion eine Sinusfunktion oder Cosinusfunktion, also eine trigonometrische Funktion. Eine Vielzahl anderer Funktionen sind möglich. Die genannte Funktion kann hierbei die Höhe oder z-Koordinate, gemessen in Richtung der Mittelachse, in Abhängigkeit von der Winkelposition um die Mittelachse sein.

Die Abgriffselemente können, wie oben beschrieben, Rotationselemente oder Walzen sein, die so eingerichtet sind, dass sie bei Rotation des Verbindungselementes um dessen Achse durch das Führungssystem, also beispielsweise durch die Bahn oder Nut, jeweils um eine eigene Rotationselementachse gedreht werden, die vorzugsweise senkrecht zur Mittelachse stehen kann. Diese Ausgestaltung ist vorteilhaft, da durch die hier stattfindende Abrollbewegung des Abgriffselements auf der Bahn oder in der Nut geringe Reibungsverluste auftreten. Insbesondere können die Abgriffselemente vorteilhaft Wälzlager (z.B. Kurvenrollen) oder Gleitlager sein, die auf der Bahn oder in der Nut abrollen, und deren Drehachsen besonders bevorzugt senkrecht zur Mittelachse sind.

Bevorzugterweise ist die Anzahl der Abgriffselement gleich der Anzahl der Lanzenaufnehmer, so dass jeder Lanzenaufnehmer durch eines der Abgriffselemente bewegbar ist.

In einer vorteilhaften Ausgestaltung der Erfindung kann der Linearisierungsmechanismus als Führungssystem auch eine Bahn oder Nut aufweisen, welche die Mittelachse mehrfach umläuft. Auf oder in dieser gleiten oder rollen die Abgriffselemente um die Mittelachse herum. Die Bahn oder Nut kann dabei verschiedene Abschnitte haben, zum einen Abschnitte mit einer Neigung eines ersten Vorzeichens, in denen sich die Bahn oder Nut dem einen Ende des Linearisierungsmechanismus in Richtung der Mittelachse nähert und zum anderen Abschnitte, mit einer Neigung des entgegengesetzten Vorzeichens, in denen sich die Bahn oder Nut dem anderen Ende des Linearisierungsmechanismus nähert. Es können dann jeweils Teilmengen der Abgriffselemente auf oder in unterschiedlichen Abschnitten der Bahn bzw. Nut gleiten oder rollen. Auf diese Weise kann eine Übersetzung der Drehzahl des Verbindungselementes erfolgen. Die Abschnitte der Bahn oder Nut können sich, sofern sie mit dem gleichen radialen Abstand zur Mittelachse und auf gleicher Höhe in Richtung der Mittelachse um diese umlaufen, kreuzen. Es ist dann vorteilhaft, wenn die Abgriffselemente in Umfangsrichtung etwas länglich ausgestaltet sind, also in Umfangsrichtung länger ausgedehnt sind, als in Richtung parallel zur Mittelachse. Diese Abgriffselemente können dann mit ihrer Längsrichtung parallel zu jenem Abschnitt der Nut oder Bahn liegen, auf oder in welcher sie gleiten oder rollen. Auf diese Weise können die Abgriffselemente an Kreuzungspunkten der Abschnitte jenem Abschnitt der Bahn oder Nut folgen, der den Abschnitt geradeaus fortsetzt, auf dem das entsprechende Abgriffselement auf den Kreuzungspunkt zuläuft. Möglich ist optional auch eine Ausgestaltung, in der der Linearisierungsmechanismus als Führungssystem zwei oder mehr Bahnen oder Nuten aufweist, die mit unterschiedlichen Vorzeichen gegenüber einer zur Mittelachse senkrechten Ebene geneigt sind. Es können dann jeweils Teilmengen der Abgriffselemente auf oder in unterschiedlichen Bahnen bzw. Nuten gleiten oder rollen. Auf diese Weise kann für die Teilmengen voneinander unabhängige Bewegungsmuster vorgegeben werden, die ihrerseits zu voneinander unabhängigen Bewegungsmustern der den entsprechenden Teilmengen entsprechenden Lanzenaufnehmern bzw. Lanzen führen.

Eine Ausgestaltung des Führungssystems mit zwei einander kreuzenden Bahnen ist besonders effizient herstellbar, indem die Bahnen oder Nuten in einem gemeinsamen Zylinder angeordnet werden. Es wird hierbei von einem gemeinsamen Zylinderkörper ausgegangen, in dem die Nuten oder Bahnen in die Zylinderfläche eingebracht werden. Die Zylinderachse steht hierbei vorzugsweise parallel zur Mittelachse.

Die Abgriffselemente können, wie oben beschrieben, mit den Lanzenaufnehmern gekoppelt sein, wobei jeweils ein Abgriffselement mit einem Lanzenaufnehmer gekoppelt sein kann. Bevorzugterweise können die Abgriffselemente bezüglich der Mittelachse radial nach außen oder radial nach innen an dem jeweiligen Lanzenaufnehmer angeordnet sein. Insbesondere, wenn die Abgriffselemente radial nach innen am Lanzenaufnehmer angeordnet sind, ist es bevorzugt, wenn der Lanzenaufnehmer entlang seines Verlaufs in Richtung der Mittelachse einen radialen Versatz nach innen aufweist, so dass jene Enden des Lanzenaufnehmers, an dem die Lanzen angeordnet werden können, dicht aneinander liegen.

In einer vorteilhaften Ausgestaltung der Erfindung kann der Linearisierungsmechanismus als Führungssystem auch einen Ring aufweisen, dessen äußerer oder innerer Rand von den Abgriffselementen umgriffen oder eingefasst wird. Vorteilhaft kann der Ring zumindest abschnittsweise gegenüber einer zur Mittelachse senkrechten Ebene geneigt sein. Es kann hier das oben zur Bahn und zur Nut Gesagte analog gelten. Insbesondere ist es vorteilhaft, wenn der Ring unterschiedlich gegenüber der Mittelachse geneigte Abschnitte aufweist. Beispielsweise kann der Ring selbst in einer Ebene verlaufen, welche Ebene jedoch nicht parallel und nicht senkrecht zur Mittelachse steht. Die Neigung des Rings als Funktion vom Winkel um die Mittelachse wäre in diesem Fall eine Sinusfunktion.

Als Ring soll hier jeder Körper angesehen werden, der entlang eines Umfangs um die Mittelachse parallel verlaufende Seitenwände aufweist und in Umfangsrichtung geschlossen ist. Vorteilhaft sind die Seitenwände vollständig durch in Umfangsrichtung geschlossene Linien oder Bahnen aufgebaut. Vorteilhaft wird jeder Körper als Ring angesehen, dessen Projektion auf eine zur Mittelachse senkrechte Ebene einen Kreisring beschreibt. Dabei ist es vorteilhaft, jedoch nicht notwendig, dass der Ring selbst in einer Ebene verläuft. Auch gebogene Ringe sind möglich.

Ist das Führungssystem ein Ring, so können die Abgriffselemente vorteilhafterweise jeweils zwei Wälz- oder Gleitlager aufweisen, die jeweils an einer der Lanzenaufnehmer angeordnet sind. Jeder Lanzenaufnehmer kann also eines der Abgriffselemente aufweisen, welches jeweils zwei der Wälz- oder Gleitlager aufweist. Bevorzugterweise stehen dabei Drehachsen der Wälzlager oder die Gleitlager jeweils senkrecht zur Mittelachse. Es kann dann jeweils eine der zwei Wälz- oder Gleitlager auf einer Seite des Rings in Richtung der Mittelachse angeordnet sein und jeweils die andere der zwei Wälz- oder Gleitlager auf der anderen, gegenüberliegenden Seite des Ringes. Der Ring liegt also dann zwischen den Wälz- oder Gleitlagern des jeweiligen Abgriffselementes. Auf diese Weise kann der Ring, wenn er um die Mittelachse gedreht wird, in beide Richtungen parallel zur Mittelachse eine Kraft auf das Abgriffselement ausüben und dieses in Richtung der Mittelachse verschieben. Dies kann dann zu einer Verschiebung des Lanzenaufnehmers und damit der Lanze führen.

In einer vorteilhaften Ausgestaltung können die Abgriffselemente jeweils eine Nut aufweisen, in welcher der äußere Rand des Ringes liegt. Der Ring kann dann in diesen Nuten jeweils gleiten. Die Nut des jeweiligen Abgriffselementes umgreift hier also den äußeren Rand des Ringes. Entsprechend können die Abgriffselemente auch den inneren Rand des Ringes umgreifen. In diesem Fall umgreifen die Abgriffselemente den Ring von innen.

In einer weiteren vorteilhaften Ausgestaltung der Erfindung kann das Führungssystem einen um die Mittelachse umlaufenden ersten Ring aufweisen, der gegenüber der Mittelachse geneigt ist, also nicht parallel und nicht senkrecht zur Mittelachse steht. Dieser Ring kann fest mit dem Verbindungselement verbunden sein. Das Führungssystem kann in dieser Ausgestaltung einen weiteren zweiten Ring aufweisen, der koaxial und bevorzugt koplanar zum ersten Ring angeordnet ist und der gegenüber dem ersten Ring beweglich gelagert ist. Es können dann die Lanzenaufnehmer jeweils mit dem zweiten Ring über jeweils zumindest ein Gelenk verbunden sein, dessen Gelenkachse bevorzugt senkrecht zur Mittelachse steht. In diesem Fall sind vorzugsweise diese Gelenke radial außerhalb des zweiten Rings angeordnet. Die Lanzenaufnehmer können hier entlang ihres Verlaufs in Richtung der Mittelachse einen radialen Versatz aufweisen, so dass sie auf ihren den Lanzen abgewandten Enden radial außerhalb des zweiten Ringes liegen und an ihren den Lanzen zugewandten Enden dichter aneinander. Der erste und der zweite Ring können auf verschiedene Weise gegeneinander gelagert sein, beispielsweise über ein Kugellager oder Gleitlager zwischen dem ersten und dem zweiten Ring.

Wird in dieser Ausführungsform das Verbindungselement gedreht, so dreht dieses den inneren ersten Ring. Der zweite Ring hingegen wird nicht um die Mittelachse gedreht. Eine Mittelsenkrechte des zweiten Ringes ist gegenüber der Mittelachse geneigt, da der erste und der zweite Ring gegenüber der Mittelachse geneigt sind. Die Mittelachse der Ringe bewegt sich dann im Verlauf der Drehung um die Mittelachse des Bohradapters herum.

Bevorzugterweise sind der erste und der zweite Ring in einer gemeinsamen Ebene angeordnet. Die Lanzenaufnehmer können dann jeweils über ein Gelenk mit einem Halteelement verbunden sein. Die Halteelemente können jeweils eine Nut aufweisen, in der ein äußerer Rand des zweiten Ringes liegt. Es ist auch möglich, dass die Halteelemente jeweils eine Sicke aufweisen, die in einer Nut im äußeren Rand des zweiten Ringes liegt. Die Nut kann hierbei parallel zum äußeren Rand des zweiten Ringes verlaufen.

In einer vorteilhaften Ausgestaltung der Erfindung ist es auch möglich, dass der erste Ring und der zweite Ring übereinander in Richtung der Mittelachse angeordnet sind. In diesem Fall können die Lanzenaufnehmer mit dem zweiten Ring jeweils über ein Scharniergelenk verbunden sein. Dieses Scharniergelenk kann zwei Schenkel miteinander verbinden, von denen ein ringseitiger Schenkel mit dem zweiten Ring verbunden ist, vorzugsweise fest verbunden ist, und von denen ein lanzenaufnehmerseitiger Schenkel mit dem entsprechenden Lanzenaufnehmer verbunden ist. Dabei ist vorzugsweise der lanzenaufnehmerseitige Schenkel mit dem entsprechenden Lanzenaufnehmer über ein weiteres Scharniergelenk verbunden. Vorteilhafterweise ist der lanzenaufnehmerseitige Schenkel außerdem in Richtung einer Drehachse des weiteren Scharniergelenks verschiebbar. Vorteilhafterweise kann die Drehachse des weiteren Scharniergelenks senkrecht zur Mittelachse stehen. Eine Drehachse des ersten oben beschriebenen Scharniergelenks kann vorteilhaft tangential zu einem parallel zum zweiten Ring verlaufenden Kreis liegen. Auch in dieser Ausgestaltung wird der erste Ring durch das Verbindungselement gedreht und bewirkt eine Drehung einer Mittelachse des zweiten Ringes um die Mittelachse des Bohradapters. Hierdurch verändert sich die Position der Lanzenaufnehmer in Richtung entlang der Mittelachse, so dass mit Drehung des Verbindungselementes die Lanzenaufnehmer entlang der Mittelachse verschoben werden.

In einer vorteilhaften Ausgestaltung der Erfindung kann das Verbindungselement an einem Ende des Verbindungselementes ein um die Verbindungselementachse verlaufendes Verbindungskegelrad aufweisen. Unter einem Kegelrad wird hier ein kegelförmiges Zahnrad verstanden.

Es kann dann der Linearisierungsmechanismus für jeden der Lanzenaufnehmer ein Abgriffskegelrad aufweisen, dessen Drehachse vorteilhaft senkrecht zur Verbindungselementachse steht. Die Abgriffskegelräder sind dann jeweils mit dem Verbindungskegelrad im Eingriff, so dass eine Rotation des Verbindungskegelrads um die Verbindungsachse zu einer Rotation der Abgriffskegelräder um deren Drehachse führt.

In dieser Ausgestaltung können die Abgriffskegelräder jeweils mit einem um die Drehachse des entsprechenden Abgriffskegelrades drehbaren Exzenter verbunden sein. Die Exzenter können ihrerseits jeweils an ihrem äußeren Rand in Richtung senkrecht zur Drehachse des entsprechenden Abgriffskegelrades mit einem Abgriffselement in Kontakt sein, das mit dem entsprechenden Lanzenaufnehmer verbunden ist. Eine Drehung des Abgriffskegelrades führt also zu einer Drehung des Exzenters, der dann das Abgriffselement verschiebt. Das Abgriffselement verschiebt dann den entsprechenden Lanzenaufnehmer entlang der Mittelachse.

Vorteilhaft kann der Exzenter eben ausgestaltet sein mit einem in der Ebene umlaufenden Rand. Die Ebene steht hierbei vorzugsweise senkrecht zur Drehachse des entsprechenden Abgriffskegelrades. Das Abgriffselement kann sich dann vorteilhaft ebenfalls in einer Ebene erstrecken, die besonders bevorzugt dieselbe Ebene ist, in der sich der Exzenter erstreckt. Die Abgriffselemente können dann jeweils in ihrer Fläche eine Vertiefung oder Öffnung aufweisen, in welcher der Exzenter angeordnet ist. Es kann dann eine entlang des Randes umlaufende Außenwand des Exzenters an einer Innenwand der Vertiefung gleiten oder abrollen. Auf diese Weise kann der Exzenter eine Verschiebung des Abgriffselements bewirken

Die Abgriffskegelräder können auch mit Kurvenscheiben verbunden sein, die eine Kurvenbahn haben, die in einer Ebene verläuft, die senkrecht zur Drehachse des entsprechenden Abgriffskegelrades steht und eine beliebige Form haben kann. Die Abgriffselemente können hier Gleit- oder Wälzlager aufweisen, die in der Kurvenbahn rollen oder gleiten. Auf diese Weise ist es möglich, durch die Form der Kurvenbahn die Bewegung der Lanzen frei zu programmieren. Insbesondere kann hierdurch eine Sinusbewegung der Lanzen mit intermittierenden Plateauphasen realisiert werden.

In einer vorteilhaften Ausgestaltung der Erfindung können die Lanzenaufnehmer jeweils einen Aufnahmekanal aufweisen, der in Richtung parallel zur Mittelachse verläuft. Es können dann die Lanzen jeweils ein Einführelement aufweisen und mit ihrem Einführelement in den Aufnahmekanal des entsprechenden Lanzenaufnehmers eingeführt werden.

Es sei angemerkt, dass die Bohrung nicht zwingend kreisförmig im Querschnitt sein muss, sondern vorteilhaft auch andere Querschnittsformen haben kann, wodurch zusätzlich gewährleistet werden kann, dass die Lanzen gegen Verdrehen um ihre Längsachse gesichert sind. Die Bohrung kann auch eine Tasche, eine Nut oder ein Schlitz sein.

Bevorzugterweise können die Aufnahmekanäle jeweils eine bezüglich der Mittelachse in radialer Richtung verlaufende erste Bohrung aufweisen. Es ist dann vorteilhaft, wenn auch die Einführelemente der Lanzen jeweils eine Bohrung, hier als zweite Bohrung bezeichnet, aufweisen, die zur ersten Bohrung koaxial liegt, wenn das Einführelement bestimmungsgemäß im entsprechenden Aufnahmekanal angeordnet ist. Es kann dann der Schnellspannmechanismus an jedem der Lanzenaufnehmer ein Verschlusselement aufweisen, das in die jeweilige erste und zweite Öffnung einschiebbar ist. Zur Anordnung der Lanzen an den Lanzenaufnehmern kann also dann die Lanze mit dem entsprechenden Einführelement in den entsprechenden Aufnahmekanal eingeschoben werden und dann das Verschlusselement in die erste und die zweite Öffnung geschoben werden.

In einer vorteilhaften Ausgestaltung der Erfindung kann der Schnellspannmechanismus außerdem eine äußere Hülle aufweisen, welche um die Lanzenaufnehmer herum angeordnet ist. Die Hülle kann also alle Lanzenaufnehmer umschließen. Die oben beschriebenen Verschlusselemente können jeweils Teil eines entsprechenden Verschlussschiebers sein, der zwischen der äußeren Hülle und dem entsprechenden Lanzenaufnehmer angeordnet sein kann. Jeder der Verschlussschieber weist also hier eines der Verschlusselemente auf. Die äußere Hülse kann dann vorteilhaft eine innere Oberfläche aufweisen, die unterschiedliche Abstände zur Mittelachse hat. Insbesondere kann die äußere Hülse einen Geöffnet-Bereich mit einem ersten Abstand von der Mittelachse aufweisen und zumindest einen Geschlossen-Bereich mit einem zweiten Abstand von der Mittelachse. Vorzugsweise ist dann der erste Abstand größer oder gleich der radialen Ausdehnung des entsprechenden Verschlussschiebers abzüglich der radialen Länge der entsprechenden ersten Bohrung. Vorzugsweise ist außerdem der zweite Abstand kleiner als die radiale Ausdehnung des entsprechenden Verschlussschiebers abzüglich der Länge der entsprechenden ersten Bohrung. Auf diese Weise ist es möglich, die Verschlusselemente durch Bewegen der äußeren Hülse in radialer Richtung zu verschieben. Ist die Hülse so positioniert, dass sich die Verschlusselemente im Geöffnet-Bereich der inneren Oberfläche der Hülse befinden, so greifen die Verschlusselemente nicht in die zweiten Bohrungen ein, so dass die Einführelemente und damit die Lanzen nicht in den Aufnahmekanälen gehalten werden. Befinden sich hingegen die Verschlusselemente im Geschlossen-Bereich der inneren Oberfläche der äußeren Hülse, so sind die Verschlusselemente in die zweite Bohrung eingeführt, wenn die Einführelemente in den Aufnahmekanälen angeordnet sind, so dass die Lanzen in den Aufnahmekanälen gehalten werden. Durch eine solche Ausgestaltung ist es möglich, die Lanzen schnell und einfach zu lösen, einzusetzen und auszutauschen.

Vorteilhafterweise kann die Hülse eine Anzahl an Geöffnet-Bereichen aufweisen, die gleich der Anzahl der Bohrlanzen ist. Außerdem kann die Hülse eine Anzahl an Geschlossen-Bereichen aufweisen, die gleich der Anzahl an Bohrlanzen ist. Es kann dann die Hülse um die Mittelachse drehbar gelagert sein und die Geöffnet-Bereiche und die Geschlossen-Bereich entlang des Umfangs der Hülse abwechselnd angeordnet sein. Auf diese Weise können die Verschlusselemente durch Drehen der Hülse vom Geöffnet- in den Geschlossen-Bereich und andersrum bewegt werden. Es wird also möglich, durch Drehen der Hülse die Lanzen zu fixieren und zu lösen.

Es ist auch möglich, dass die Hülse einen der Geöffnet-Bereiche und einen der Geschlossen-Bereiche aufweist, wobei der Geöffnet-Bereich und der Geschlossen-Bereich in Richtung der Mittelachse hintereinander angeordnet sind und vorzugsweise die Hülse auf ihren gesamten Umfang umlaufen. Es kann dann die Hülse in Richtung der Mittelachse verschiebbar sein. In dieser Ausgestaltung ist es möglich, die Verschlusselemente vom Geöffnet-Bereich in den Geschlossen-Bereich dadurch zu bewegen, dass die Hülse in Richtung der Mittelachse verschoben wird. Auch hierdurch ist es auf einfach Weise möglich, die Lanzen an den Lanzenaufnehmern zu fixieren und zu lösen.

Vorteilhafterweise kann der Schnellspannmechanismus außerdem ein Gehäuse aufweisen, das die Hülse umgibt. Die Hülse kann dann auf ihrer Oberfläche zumindest einen Knopf aufweisen. Das Gehäuse kann hier eine Führungsnut aufweisen, durch welche der zumindest eine Knopf hindurchtritt. Die Führungsnut kann vorteilhaft einen Abschnitt aufweisen, der parallel verläuft zu einer Richtung, in welcher die Hülse verschiebbar ist, um die Verschlusselemente in die zweiten Öffnungen einzuführen und aus den zweiten Öffnungen zu entfernen. Es kann daher durch Betätigung des zumindest einen Knopfes von außen die Hülle verschoben werden und damit die Lanzen gelöst oder befestigt werden.

In einer weiteren vorteilhaften Ausgestaltung der Erfindung können die Lanzenaufnehmer jeweils eine erste Ausnehmung in radialer Richtung nach außen aufweisen. Darüberhinaus können die Einführelemente jeweils eine zweite Ausnehmung in radialer Richtung nach außen aufweisen. Die erste Ausnehmung und die zweite Ausnehmung sind jeweils so angeordnet, dass sie im Zustand, in dem die Lanzen bestimmungsgemäß in die Einführelemente eingeführt sind, auf gleicher Höhe in Richtung der Mittelachse ausgebildet sind. Es kann dann der Bohradapter außerdem eine der Anzahl der Bohrlanzen gleiche Anzahl an Verschlussschiebern aufweisen, die sich in radialer Richtung erstrecken von einem Abstand zur Mittelachse, der größer oder gleich ist als ein minimaler Abstand der radial äußeren Oberfläche der Einführelemente von der Mittelachse im Bereich der zweiten Ausnehmungen zu einem Abstand zur Mittelachse, der größer ist als ein Abstand einer radial außenliegenden Oberfläche der Lanzenaufnehmer außerhalb der ersten Ausnehmungen. Die Verschlussschieber können also eine solche radiale Ausdehnung aufweisen, dass sie von außerhalb der ersten Ausnehmungen kontaktierbar sind und sich bis in die zweiten Ausnehmungen hinein erstrecken, wenn sie über den ersten und zweiten Ausnehmungen angeordnet sind.

Der Bohradapter kann dann eine Konturhülse aufweisen, welche die Lanzenaufnehmer umgibt und die auf ihrer inneren Oberfläche eine Anzahl an Führungsnuten aufweist, die gleich der Anzahl der Lanzen ist. Die Führungsnuten können dabei parallel zur Mittelachse verlaufen und die besagten Verschlussschieber können jeweils in einer der Führungsnuten der Konturhülse angeordnet sein. Die Verschlussschieber können also durch die Führungsnuten in Richtung parallel zur Mittelachse geführt werden. Eine Länge der Führungsnuten in Richtung parallel zur Mittelachse ist dabei vorzugsweise größer oder gleich einer Amplitude der oszillierenden Bewegung der Bohrlanzen.

In dieser Ausgestaltung können die Verschlussschieber durch Rotation der Hülse in die ersten und zweiten Ausnehmungen eingeführt werden und aus diesen herausgeschoben werden. Hierdurch können die Lanzen befestigt und gelöst werden.

Die Verschlussschieber sind vorteilhaft immer mit den Lanzenaufnehmern in Kontakt. Mit den Einführelementen der Lanzen sind sie hingegen nur im fixierten Zustand in Kontakt. Durch Rotation der Hülse können die Verschlussschieber zwischen diesen Positionen verschoben werden.

In einer vorteilhaften Ausgestaltung der Erfindung können die Einführelemente der Lanzen jeweils einen Vorsprung aufweisen. Die Aufnahmekanäle der Lanzenaufnehmer können dann jeweils eine Nut in ihrer inneren Oberfläche aufweisen, die von einem den Lanzen zugewandten Rand, also von dem dem Verbindungselement abgewandten Rand, des entsprechenden Aufnahmekanals zunächst parallel zur Mittelachse verläuft und dann in einer Kurve um einen Winkel von 90° oder mehr abknickt. Der Vorsprung und die Nut sind dabei so bemessen, dass beim Einführen der Einführelemente der Lanzen in die Aufnahmekanäle der jeweilige Vorsprung durch die jeweilige Nut geführt wird. Die Einführelemente sind dann also in die Aufnahmekanäle einführbar und wenn die Vorsprünge an der Kurve der Nut angekommen sind, kann die Lanze um einige Grad um ihre Längsachse oder die Mittelachse gedreht werden und ist dadurch in dem Aufnahmekanal arretierbar.

In einer weiteren vorteilhaften Ausgestaltung der Erfindung können die Aufnahmekanäle auf ihrer inneren Oberfläche jeweils einen Vorsprung aufweisen und es können die Einführelemente auf ihrer äußeren Oberfläche jeweils eine Nut aufweisen. Dabei ist die innere Oberfläche jene der Mittelachse zugewandte Oberfläche und die äußere Oberfläche jeweils die der Mittelachse abgewandte Oberfläche. Die Nuten in den Einführelementen können dann jeweils von einem einer Bearbeitungsspitze der entsprechenden Lanze abgewandten Rand des Einführelementes zunächst parallel zur Mittelachse verlaufen und dann in einer Kurve um einen Winkel von 90° oder mehr abknicken. Es ist dann der Vorsprung der Aufnahmekanäle jeweils beim Einführen der entsprechenden Lanze in der Nut des entsprechenden Einführelementes führbar. Auch hier sind die Lanzen dadurch arretierbar, dass sie um die jeweilige Längsachse der Lanze oder die Mittelachse gedreht werden. In den Ausgestaltungen mit Vorsprüngen und abgeknickten Nuten können die Lanzen ohne äußere Hülse arretiert werden, was die Zahl der Bauteile verringern kann.

In einer weiteren vorteilhaften Ausgestaltung der Erfindung können die Einführelemente jeweils an ihrem der Bohrlanzenspitze abgewandten Ende einen Schnapphaken aufweisen. Es können dann die Lanzenaufnehmer jeweils eine radiale Durchgangsöffnung auf jener Höhe in Richtung der Mittelachse aufweisen, wo der Schnapphaken des entsprechenden Einführelementes in einem Zustand, in dem die Einführelemente bestimmungsgemäß in die Aufnahmekanäle eingeführt sind, eine Haltefläche aufweist. Die Schnapphaken können dann in diese Durchgangsöffnung einrasten. In jeder der Durchgangsöffnungen kann dann ein Stift vorgesehen sein, der in Richtung der Längsachse der entsprechenden Durchgangsöffnung verschiebbar ist. Zum Lösen der Lanzen können dann die Stifte in radialer Richtung nach innen gedrückt werden, wodurch der Schnapphaken befreit wird und die Lanze gelöst wird. Zum Drücken der Stifte kann in radial äußerer Richtung ein Knopf vorgesehen sein, der von außen manuell drückbar ist. Durch Drücken des Knopfes kann also der Stift nach radial innen gedrückt werden und dadurch die Lanze befreien. Vorteilhaft kann der Knopf so gefedert gelagert sein, dass er bei Abwesenheit äußerer Kräfte den nicht auf den entsprechenden Stift drückt. Die Stifte können in der Durchgangsöffnung frei beweglich gleiten und werden vom Schnapphaken verdrängt, wenn dieser in die Durchgangsöffnung einrastet.

Vorteilhafterweise kann der Bohradapter in dieser Ausgestaltung auch ein die Lanzenaufnehmer umgebendes Gehäuse aufweisen. Es kann dann für jeden der Stifte ein Rückhalteelement vorgesehen sein, an dem der entsprechende Stift mit seinem radial äußeren Ende anliegt. Die Rückhalteelemente können jeweils durch eine radiale Öffnung in einer Wand des Gehäuses verlaufen und außerhalb des Gehäuses mit einem Griff oder Knopf verbunden sein. Innerhalb des Gehäuses können sie gegen eine Innenoberfläche der Gehäusewand federnd abgestützt sein. Die Rückhalteelemente können dann jeweils eine Gleitfläche aufweisen, an der der entsprechende Stift anliegt. Eine Erstreckung der Gleitflächen in Richtung parallel zur Mittelachse ist vorteilhaft größer oder gleich einer Amplitude der oszillierenden Bewegung der Bohrlanzen. Bei Drehung des Verbindungselementes und damit bei Bewegung der Lanzenaufnehmer gleitet der Stift mit seinem radial äußeren Ende an dem Rückhalteelement, wird durch dieses aber in Kontakt mit dem entsprechenden Schnapphaken gehalten. Die hierzu erforderliche Kraft in radialer Richtung kann durch die Feder bewirkt werden. Durch Betätigen des Griffs oder des Knopfes kann der Stift gedrückt werden und dadurch die Lanze lösen.

In einer weiteren vorteilhaften Ausgestaltung können die Lanzenaufnehmer einen gemeinsamen Kanal umschließen, der koaxial zur Mittelachse liegt. Der Kanal kann dann ein Innengewinde aufweisen, das ebenfalls koaxial zur Mittelachse ist. Die Bohrlanzen können hierbei jeweils ein Einführelement aufweisen, wobei die Einführelemente aller der Bohrlanzen zusammen ein gemeinsames Außengewinde zusammensetzen. Es sind dann die Einführelemente gemeinsam mit diesem Außengewinde in das gemeinsame Innengewinde der Lanzenaufnehmer einschraubbar. Diese Ausgestaltung ermöglicht es besonders vorteilhaft, alle Lanzen gemeinsam als Satz in dem Lanzenaufnehmer anzuordnen und aus diesem zu entfernen. Der Kanal kann an seinem den Bohrlanzen zugewandten Ende eine lineare Gleitführung aufweisen, in der die Lanzen gleiten können. Hier ist es vorteilhaft, die Lanzen vor dem Bohren gegen Verdrehen zu sichern, damit sie sich nicht von selbst lösen können. Hierzu kann eine Kappe mit einem Innengewinde vorgesehen sein, die auf ein Außengewinde eines Gehäuses aufschraubbar sein kann, in dem vorteilhaft der Schnellspannmechanismus untergebracht ist. Die Kappe kann die lineare Gleitführung für die Lanzen durch Kraftschluss am Gehäuse fixieren, so dass diese sich nicht verdrehen kann. Vorteilhaft können hier die Lanzen eingesteckt und dann noch einmal verdreht werden. Die vordere Gleitführung (welche optional aber vorteilhaft ist) kann sich dann vorteilhaft mitdrehen. Diese frontale Gleitführung wird dann durch das Anpressen der Kappe kraftschlüssig fixiert und so gegen verdrehen gesichert. Damit können sich auch die Lanzen nicht mehr drehen und somit nicht von selbst lösen.

In einer weiteren möglichen Ausgestaltung der Erfindung können die Lanzenaufnehmer jeweils einen Schlitz radial nach außen aufweisen, der eine den entsprechenden Aufnahmekanal umschließende Wand durchstößt. Die entsprechende Wand des Lanzenaufnehmers ist also nach außen geschlitzt. Es können dann die Einführelemente so bemessen sein, dass sie kraftschlüssig im entsprechenden Aufnahmekanal gehalten werden. Vorteilhaft kann der Schnellspannmechanismus für jeden der Lanzenaufnehmer dann ein keilförmiges Spreizelement aufweisen, das in radialer Richtung verschiebbar gelagert ist und das eine in Richtung der Mittelachse orientierte Kante aufweist. Die Kante kann in radialer Richtung über dem entsprechenden Schlitz angeordnet sein. Vorzugsweise kann das Spreizelement mittels einer Feder in einer Position gehalten werden, die beabstandet ist vom entsprechenden Schlitz. Das Spreizelement kann dann gegen die Federkraft verschiebbar sein, und zwar dergestalt, dass die Kante in den Schlitz verschiebbar ist. Durch Drücken der Kante in den Schlitz kann dieser gespreizt werden und dadurch das Einführelement und damit die entsprechende Lanze freigegeben werden. Wird das Spreizelement nicht betätigt, so wird der Schlitz nicht gespreizt und die Innenwand des Aufnahmekanals übt eine haltende Kraft auf das Einführelement der entsprechenden Lanze aus.

Das beschriebene Verbindungselement kann vorteilhaft eine Welle sein oder eine Manschette, eine Schelle oder eine Klemme. Eine solche Welle kann beispielsweise in ein Bohrfutter einer Handbohrmaschine, einer Standbohrmaschine, einer Fräsmaschine oder einer Drehmaschine eingespannt werden. Ist das Verbindungselement eine Schelle oder eine Klemme, so können diese außen an einen drehenden Teil des Bohrfutters anlegbar sein. Die Verbindung zwischen dem Bohrfutter und der Welle sollte dabei so herstellbar sein, dass ein Drehmoment von dem Bohrfutter auf das Verbindungselement übertragbar ist. In einer bevorzugten Ausgestaltung der Erfindung liegt die Achse des Verbindungselementes, also dessen Drehachse, koaxial zur Mittelachse. Der erfindungsgemäße Bohradapter kann vorteilhaft mit jeder Form rotierenden Antriebs betrieben werden, insbesondere wenn die Bestimmung des Geräts zur spanenden Bearbeitung dient, da dann der Antrieb genügend Kraft hat.

Die Verwendung von Bohrlanzen gemäß der vorliegenden Erfindung ermöglicht es, Löcher beliebiger Geometrien zu bohren. Dabei wird ein Querschnitt der erzeugten Bohrung durch den Querschnitt der Lanzen bestimmt. Vorteilhafterweise liegen die Bohrlanzen mit parallelen Längsachsen der Bohrlanzen aneinander an und bilden bei Anordnung auf gleicher Höhe zusammen eine Spitze, die einen kreisförmigen, rechteckigen, dreieckigen, sechseckigen, achteckigen, sternförmigen oder anders geformten Umfang aufweist. Da die Bohrlanzen bestimmungsgemäß entlang ihrer Längsachsen gegeneinander verschoben werden, soll die Umfangsform in einem Zustand bestimmt sein, in dem die Bohrlanzen auf gleicher Höhe angeordnet sind.

Erfindungsgemäß wird außerdem ein Bohrsystem angegeben, das einen Bohradapter wie vorstehend beschrieben aufweist und darüberhinaus eine Antriebsvorrichtung, die mit dem Verbindungselement verbunden ist, so dass ein Drehmoment von der Antriebsvorrichtung auf das Verbindungselement übertragbar ist. Vorteilhafterweise kann die Antriebsvorrichtung beispielsweise eine Handbohrmaschine, eine Standbohrmaschine, eine Fräsmaschine oder eine Drehmaschine sein.

Bevorzugterweise weist dieses Bohrsystem außerdem eine Fixierungsvorrichtung auf, die sowohl mit dem Bohradapter als auch mit der Antriebsvorrichtung verbunden ist und als Drehmomentstütze gegen die Drehung des Verbindungselementes wirkt. Eine solche kann beispielsweise eine Stange sein, die an einem Gehäuse der Antriebsvorrichtung und einem Gehäuse des Bohradapters unbeweglich angeordnet ist. Ist die Antriebsvorrichtung eine Handbohrmaschine, so kann die Stange auch mit einem Zusatzgriff zum Halten der Bohrmaschine verbunden sein und an diesem fixiert sein. Alternativ sind auch Schellen oder Klemmen möglich, die an feststehenden Teilen des Gehäuses des Antriebs befestigt werden.

Die Ausführung als auf Bohrmaschinen aufsetzbarer Adapter ermöglicht einen günstigeren Vertrieb, da kein vollständig neues Bohrsystem (inkl. Antrieb etc.) angeschafft werden muss und senkt so Markteintrittshürden.

Die Erfindung beschreibt zum ersten Mal ein System, welches gezielt dafür bestimmt ist, auf bestehende Antriebsmaschinen (z.B. Bohrmaschinen etc.) aufgesetzt zu werden und weist die hierfür erforderlichen Vorrichtungen auf (Ein wichtiger Punkt hierbei ist auch, dass der Adapter sehr schnell aufgesetzt und fixiert werden kann ohne größere Montage/Demontagearbeiten). Dieses System braucht selbst keinen Antrieb, ist also vielseitiger einsetzbar und für die verarbeitende Industrie leichter nachrüstbar.

Die Existenz eines funktionellen Schnellspannsystems macht die Pendelhubbohrtechnologie erst wirklich attraktiv für den Heimwerkerbereich und das Handwerk. Müssten die Bohrwerkzeuge alternativ in minutenlanger Arbeit getauscht werden, würde das die Attraktivität beim Kunden stark mindern.

Im Folgenden soll die Erfindung anhand einiger Figuren beispielhaft erläutert werden. Gleiche Bezugszeichen kennzeichnen dabei gleiche oder entsprechende Merkmale.

Es zeigt:
- Figur 1: eine perspektivische Ansicht eines erfindungsgemäßen Bohrersystems,
- Figur 2: eine erste vorteilhafte Ausgestaltung eines erfindungsgemäßen Bohradapters,
- Fig. 3: eine weitere vorteilhafte Ausgestaltung des erfindungsgemäßen Bohradapters,
- Fig. 4: eine weitere vorteilhafte Ausgestaltung des erfindungsgemäßen Bohradapters,
- Figur 5: eine weitere vorteilhafte Ausgestaltung des erfindungsgemäßen Bohradapters,
- Figur 6: eine weitere vorteilhafte Ausgestaltung eines erfindungsgemäßen Bohradapters,
- Figur 7: einen Teil eines Linearisierungsmechanismus mit mehreren Bahnen,
- Figur 8: eine weitere vorteilhafte Ausgestaltung eines erfindungsgemäßen Bohradapters,
- Figur 9: eine weitere vorteilhafte Ausgestaltung eines erfindungsgemäßen Bohradapters,
- Figur 10 und Figur 11: eine weitere vorteilhafte Ausgestaltung eines erfindungsgemäßen Bohradapters,
- Figur 12: eine weitere vorteilhafte Ausgestaltung eines erfindungsgemäßen Bohradapters,
- Figur 13: eine weitere vorteilhafte Ausgestaltung eines erfindungsgemäßen Bohradapters,
- Figur 14: eine weitere vorteilhafte Ausgestaltung eines erfindungsgemäßen Bohradapters,
- Figur 15: eine weitere vorteilhafte Ausgestaltung eines erfindungsgemäßen Bohradapters,
- Figur 16: eine weitere vorteilhafte Ausgestaltung eines erfindungsgemäßen Bohradapters,
- Figur 17: eine weitere vorteilhafte Ausgestaltung eines erfindungsgemäßen Bohradapters,
- Figur 18: ein Beispiel einer Bohrlanze,
- Figur 19 und Figur 20: eine beispielshafte Ausgestaltung eines Schnellspannmechanismus,
- Figuren 21, 22 und 23: eine weitere beispielhafte Ausgestaltung eines Schnellspannmechanismus,
- Figur 24 und Figur 25: eine weitere beispielhafte Ausgestaltung eines Schnellspannmechanismus,
- Figur 26 und Figur 27: ein Beispiel für Bohrlanzen,
- Figur 28: ein weiteres Beispiel eines Schnellspannmechanismus,
- Figur 29: eine beispielhafte Ausgestaltung einer Bohrlanze zur Anwendung im Schnellspannmechanismus der Figur 28,
- Figur 30: ein weiteres Beispiel eines Schnellspannmechanismus und
- Figur 31: ein weiteres Beispiel eines Schnellspannmechanismus.

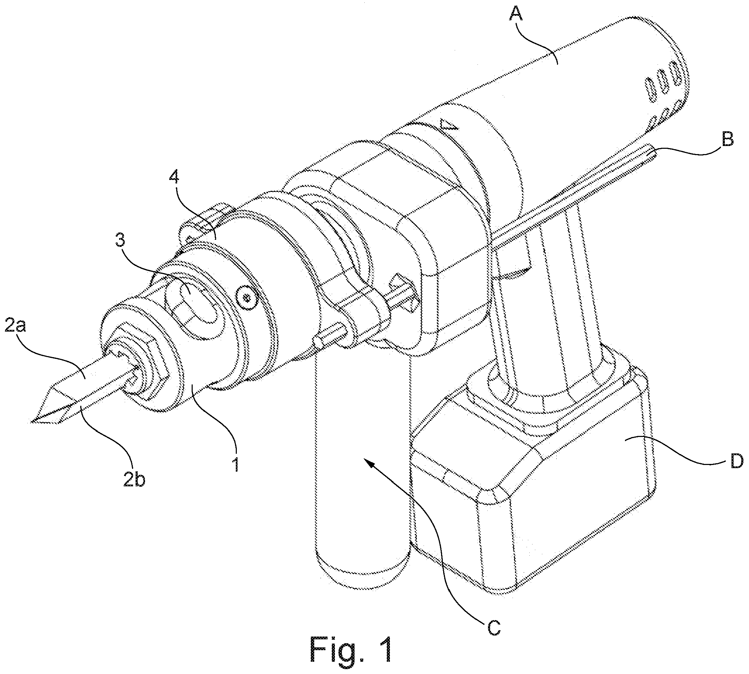

Figur 1 zeigt perspektivisch ein Beispiel eines erfindungsgemäßen Bohrsystems, das zum einen einen erfindungsgemäßen Bohradapter 1 und zum anderen eine Antriebsvorrichtung A aufweist. Die Antriebsvorrichtung ist im gezeigten Beispiel eine Handbohrmaschine A mit einem Akku D. Zwischen dem Antriebsmechanismus A und dem Bohradapter 1 ist ein Handgriff C vorgesehen, der als Teil der Handbohrmaschine A oder als Teil des Bohradapters 1 angesehen werden kann. Das in Figur 1 gezeigte Bohrsystem weist außerdem eine Fixierungsvorrichtung B auf, die als Drehmomentstütze wirkt. Diese ist zum einen mit der Antriebsvorrichtung A und/oder dem Handgriff C unbeweglich verbunden und zum anderen mit dem Bohradapter 1. Die Fixierungsvorrichtung B ist also unbeweglich gegenüber dem Handgriff C, dem Gehäuse der Antriebsvorrichtung A und einem äußeren Gehäuse des Bohradapters 1.

Das in Figur 1 gezeigte Bohrsystem weist Bohrlanzen 2a, 2b auf, die aneinander anliegend mit parallelen Längsachsen angeordnet sind und an ihrem dem Bohradapter 1 abgewandten Ende eine gemeinsame Spitze formen. Im in Figur 1 gezeigten Beispiel sind drei Bohrlanzen vorgesehen, von denen zwei Bohrlanzen 2a, 2b zu sehen sind. Eine Gerade, die durch die sich treffenden Spitzen der Bohrlanzen 2a, 2b verläuft und koaxial ist zu einer Achse, um die eine in der Figur 1 nicht zu erkennende Welle des Antriebsmechanismus A dreht, soll hier als Mittelachse angesehen werden. Die Bohrlanzen 2a, 2b sind um diese Mittelachse herum angeordnet. Der Bohradapter 1 weist einen Linearisierungsmechanismus 4 auf, mit dem eine Rotation der Welle des Antriebs A in eine oszillierende Bewegung der Bohrlanzen 2a, 2b in Richtung parallel zur Mittelachse überführbar ist. Der Bohradapter 1 weist außerdem einen Schnellspannmechanismus 3 auf, mit dem die Bohrlanzen an dem Bohradapter 1 befestigbar sind.

Die Antriebsvorrichtung A kann auch ein Akkubohrer, ein Akkuschrauber, eine Schlagbohrmaschine, eine Bohrmaschine mit Netzkabel, eine stationäre Bohrmaschine, wie beispielsweise eine Ständerbohrmaschine, Säulenbohrmaschine, Tischbohrmaschine, ein Fräser, eine Drehmaschine, ein Erdbohrer oder ein anderer Antrieb zur spanenden Fertigung sein. Durch den Linearisierungsmechanismus 4 kann die durch eine solche Antriebsvorrichtung A erzeugte Drehbewegung in eine Pendelhubbewegung der Bohrlanzen 2a, 2b überführt werden. Die Bohrlanzen 2a, 2b können auch als Bohrraspeln oder Bohrfeilen ausgestaltet sein.

Der Umfang der Gesamtheit der Bohrlanzen 2a, 2b in einer Ebene senkrecht zur Mittelachse bestimmt den Querschnitt der Löcher, die mit dem erfindungsgemäßen Bohrsystem erzeugt werden können. Hier sind beispielsweise eckige oder runde Querschnitte möglich. Jede andere Querschnittsform kann so ebenfalls erzeugt werden.

Die Fixierungsvorrichtung B, hier im Beispiel eine handelsübliche Stange, dient dazu, den Bohradapter 1 verdrehsicher an der Bohrmaschine A zu fixieren. Sie kann zum einen am Gehäuse des Bohradapters 1 verankert sein und zum anderen am Handgriff C und/oder an der Bohrmaschine A selbst. Es ist alternativ auch möglich, das Gehäuse des Bohradapters 1 mittels anderer feststehender Teile mit dem Gehäuse des Antriebs A zu verbinden. Dies können beispielsweise Schellen und/oder Klemmen um das Gehäuse des Bohradapters 1, am Griff C oder an sonstigen Teilen der Gehäuse sein. Diese Schellen/Klemmen können über eine Stange verdrehsicher mit dem Bohradapter 1 verbunden sein. Optional kann die Stange B eine feststellbare Längeneinstellung aufweisen. Bei Verwendung von Säulen-/Ständerbohrmaschinen als Antriebsvorrichtung A können Klemmen und/oder Schellen auch um eine feststehende Bohrspindelaufnahme angeordnet werden. Auch kann eine solche Verbindung zum Anschlag, einer Gehäuseaufnahme eines Vorschubhebels/Führungshebels, einem Tisch, einer Halterung einer Schutzabdeckung (bei modernen Maschinen), einer Säule oder einem anderen Teil der Bohrmaschine hergestellt werden. Hierfür ist eine zweifache (zwei Koordinatenrichtungen) feststellbare Längeneinstellung des Gestänges vorteilhaft, da die Bohrmaschinen unterschiedlich groß sein können. Außerdem ist es vorteilhaft, wenn eine Verschieblichkeit in Bohrrichtung gewährleistet ist, da sich der Adapter relativ zum Gehäuse (und den darin verankerten Teilen) bewegen kann. Ähnliche Verbindungen können auch bei Dreh- und/oder Fräsmaschinen zum Einsatz kommen.

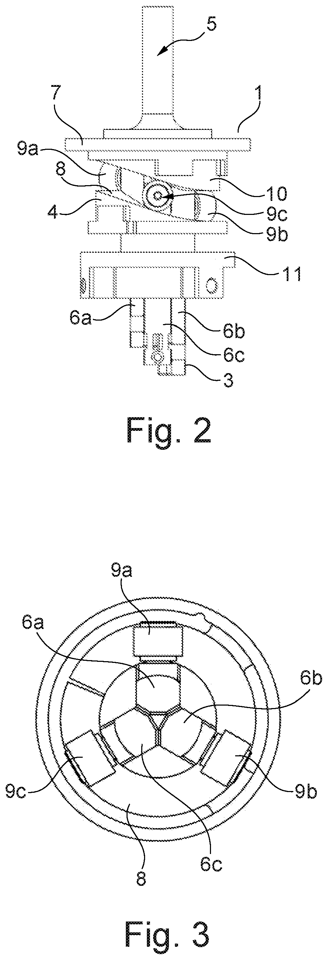

Figur 2 zeigt ein Beispiel eines erfindungsgemäßen Bohradapters 1, jedoch ohne umgebendes Gehäuse. Der Bohradapter 1 weist hier ein Verbindungselement 5 auf, das um eine im Folgenden als Verbindungselementachse bezeichnete Achse drehbar ist. Im gezeigten Beispiel liegt die Verbindungselementachse koaxial zur Mittelachse. Das Verbindungselement 5 ist hier eine Welle 5. Das Verbindungselement 5 wird dann mit dem rotierenden Teil des Antriebsmechanismus A verbunden. Es ist in allen Ausführungsformen der Erfindung vorgesehen, dass das Verbindungselement stets mit dem rotierenden Teil des Antriebsmechanismus verbunden wird oder verbindbar ist.

Ist der Antriebsmechanismus A eine Bohrmaschine, so kann das Verbindungselement 5 mit einem Schnellspanner eines Bohrfutters der Bohrmaschine verbunden werden. Alternativ kann auch eine Grundfläche 7 als Verbindungselement fungieren. In diesem Fall kann die Verbindung mit dem rotierenden Teil des Antriebsmechanismus beispielsweise über eine Schelle hergestellt werden. Die Welle 5 ist in diesem Fall nicht erforderlich. Auch andere Spannmechanismus können die Verbindung zwischen dem Antriebsmechanismus A und dem Verbindungselement 5 oder 7 herstellen.

Der Bohradapter 1 weist zwei funktionale Einheiten auf, nämlich zum einen den Schnellspannmechanismus 3 und zum anderen den Linearisierungsmechanismus 4. Es sei ausdrücklich darauf hingewiesen, dass alle Beispiele, in denen der Linearisierungsmechanismus 4 beschrieben wird, mit allen Beispielen kombiniert werden können, in denen der Schnellspannmechanismus 3 im Detail gezeigt wird.

Im in Figur 2 gezeigten Beispiel soll davon ausgegangen werden, dass das Verbindungselement 5 die Welle 5 ist. Die Welle 5 ist mit der Grundfläche 7 verbunden, die sich zusammen mit der Welle 5 dreht. Auf der Grundfläche 7 ist eine Zylinderfläche 10 mit zur Mittelachse koaxialer Zylinderachse angeordnet, in der eine Nut 8 eingebracht ist. Die Nut 8 umläuft die Zylinderachse und die Mittelachse in der Zylinderwand 10 mit unterschiedlichen Steigungen. Vorteilhaft ist hier zum Beispiel eine Form, in der die Steigung der Nut 8 abschnittsweise einer Sinusfunktion folgt, die jedoch an ihren Extrema mit Plateaus, also Bereichen mit Steigung Null, abgeflacht ist, oder bei der Plateaus zwischen ganzen oder halben Phasen der Sinusfunktion anschließend an ein Extremum vorgesehen sind.

In der Nut 8 rollen oder gleiten Abgriffselemente 9a, 9b, 9c. Die Abgriffselemente 9a, 9b, 9c können als Wälz- oder Gleitlager ausgebildet sein, die um eine zur Mittelachse senkrechte Achse rollen. Im gezeigten Beispiel stehen die Rollenachsen der Wälzlager 9a, 9b, 9c jeweils in einem Winkel von 120° zueinander um die Mittelachse.

Wird das Verbindungselement 5 um die Mittelachse angetrieben, so wird der Zylinder 10 um die Mittelachse gedreht. Durch Wechselwirkung mit der Nut werden hierdurch die Abgriffselemente 9a, 9b, 9c in Richtung der Mittelachse verschoben. Der zeitliche Verlauf der Verschiebung der Abgriffselemente 9a, 9b, 9c wird durch die Form der Nut bestimmt.

Jedes der Abgriffselemente 9a, 9b, 9c ist mit einem Lanzenaufnehmer 6a, 6b, 6c verbunden. Beispielsweise kann die Drehachse des jeweiligen Abgriffselements 9a, 9b, 9c mit dem entsprechenden Lanzenaufnehmer 6a, 6b, 6c verbunden sein. Vorzugsweise steht die Drehachse dabei senkrecht auf der Längsache des entsprechenden Lanzenaufnehmers 6a, 6b, 6c.

Vom entsprechenden Lanzenaufnehmer 6a, 6b, 6c aus gesehen weist die entsprechende Drehachse des entsprechenden Abgriffselementes 9a, 9b, 9c bezüglich der Mittelachse nach außen. Hierdurch ist es möglich, die Lanzenaufnehmer 6a, 6b, 6c unmittelbar aneinander anliegend anzuordnen.

Die Bohrlanzen 2a, 2b, 2c können an jenem dem Verbindungselement 5 abgewandten Ende der Lanzenaufnehmer 6a, 6b, 6c angeordnet werden. Hierfür ist ein Schnellspannmechanismus 3 vorgesehen, mit dem die Lanzen 2a, 2b, 2c an der entsprechenden Lanzenaufnehmern 6a, 6b, 6c angeordnet werden können. Der eigentliche Schnellspannmechanismus 3 ist in Figur 2 nicht eingezeichnet. Diesbezüglich wird auf die weiter unten stehende Beschreibung verwiesen.

Figur 3 zeigt eine Ansicht des in Figur 2 gezeigten Bohradapters 1 von oben, also aus Richtung des Verbindungselementes 5 in Richtung der Lanzenaufnehmer 6a, 6b, 6c. Das Verbindungselement 5 selbst sowie die Grundfläche 7 wurden hierbei entfernt. In den Beispielen sind wie oben beschrieben drei Lanzenaufnehmer 6a, 6b, 6c vorgesehen, die aneinander anliegen und nach außen jeweils mit einem Abgriffselement 9a, 9b, 9c verbunden sind. Die Abgriffselemente laufen in der Nut 8, von der hier die untere Seitenfläche gezeigt ist.

Der in Figur 2 gezeigte Zylinder kann als eine Art Kurventrommel mit innenlaufender Bahn oder Nut 8 angesehen werden. Zwei einfassende Teile dieser Kurventrommel 10, welche die Bahn 8 zusammenhalten, sind hier nicht dargestellt. Durch Rotation der Kurventrommel um die Mittelachse bewegen sich die Abgriffselemente 9a, 9b, 9c, hier als Kurvenrollen 9a, 9b, 9c ausgestaltet entsprechend der Bahn auf und ab. Anstelle der Kurvenrollen können auch Wälzlager oder Gleitlager vorgesehen sein.

Die Bahn oder Nut 8 kann beispielsweise eine trigonometrische, polynomiale oder auch lineare Form entlang ihres Verlaufs um die Mittelachse herum aufweisen. Diese kann jeweils kontinuierlich oder mit Plateauphasen ausgestaltet sein. Die Bahn bzw. Nut 8 gibt dabei das zeitabhängige bzw. zyklusabhängige Ausmaß der Auf- und Abbewegung an.

Die Abgriffselemente 9a, 9b, 9c sind mit den Lanzenaufnehmern 6a, 6b, 6c verbunden, die im gezeigten Beispiel in einer Linearführung 11 verdrehsicher geführt werden, so dass nur eine Bewegung in Richtung der Mittelachse möglich ist. Auf diese Weise bewegen sich die Lanzenaufnehmer 6a, 6b, 6c und damit die an diesen verbundenen Bohrlanzen 2a, 2b, 2c nacheinander zyklisch in definiertem Maß auf und ab.

Die Winkelanordnung der Abgriffselemente 9a, 9b, 9c zueinander bestimmt die Phasenbeziehung der Bewegungen der Bohrlanzen 2a, 2b, 2c zueinander. Sind die Abgriffselemente 9a, 9b, 9c gleichmäßig um die Mittelachse verteilt, so ist im Allgemeinen der Winkel zwischen den Drehachsen zwei benachbarter Abgriffselemente 9a, 9b, 9c gleich 360° geteilt durch die Anzahl der Abgriffselemente 9a, 9b, 9c oder der Anzahl der Bohrlanzen 2a, 2b, 2c.

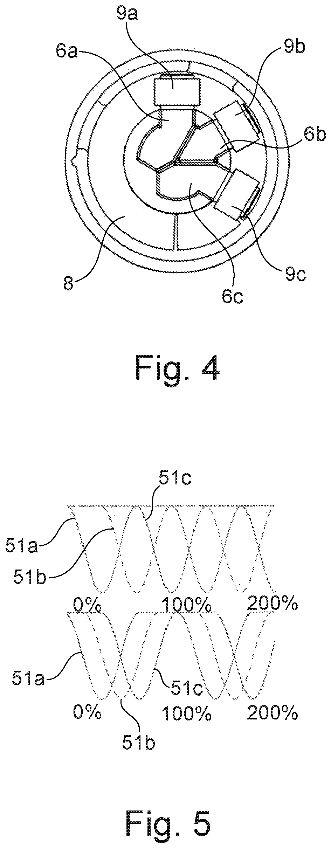

Figur 4 zeigt eine alternative Winkelanordnung der Abgriffselemente 9a, 9b, 9c um die Mittelachse. Während die Abgriffselemente 9a, 9b, 9c im in Figur 3 gezeigten Beispiel mit gleichen Winkeln voneinander beabstandet angeordnet sind, ist dies in Figur 4 nicht der Fall. In Figur 4 sind die Winkel zwischen den Abgriffselementen 9a und 9b sowie zwischen den Abgriffselementen 9b und 9c gleich groß und deutlich kleiner als der Winkel zwischen den Abgriffselementen 9a und 9c. Die Lanzenaufnehmer 6a, 6b, 6c sind hier so ausgestaltet, dass sie diese unterschiedlichen Winkel realisieren, jedoch dennoch um die Mittelachse aneinander anliegen.

Figur 5 zeigt die Position der Lanzenaufnehmer 6a, 6b, 6c (vertikale Richtung des Diagramms) in Abhängigkeit von der Winkelposition des Verbindungselements 5 um die Mittelachse. Dabei zeigt das obere Diagramm den Verlauf bei einer Anordnung der Abgriffselemente wie in Figur 3 und das untere Diagramm einen Verlauf bei Anordnung der Abgriffselemente wie in Figur 4. Durch die Anordnung wie in Figur 4 werden die zeitlichen Abstände der einzelnen Bewegungen der Bohrlanzen im Zyklus verringert. Der obere Zyklus in Figur 5 ist gleichmäßig mit Plateauphasen, während der untere Zyklus ungleichmäßig mit Plateauphasen ist. Die Linearführungen und die Form der Lanzenaufnehmer 6a, 6b, 6c ist dementsprechend konstruktiv angepasst. Das Prinzip der ungleichmäßigen Verteilung über den Bewegungszyklus kann auf alle Varianten der Erfindung übertragen werden. Es soll im Folgenden jedoch zum besseren Verständnis nur der Fall gleichmäßig verteilter Abgriffselemente gezeigt werden.

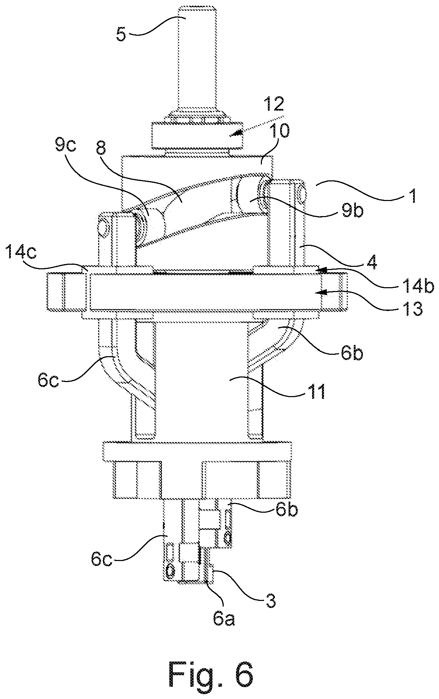

Figur 6 zeigt ein weiteres Beispiel eines erfindungsgemäßen Bohradapters 1, wobei auch hier nur der Linearisierungsmechanismus 4 gezeigt ist, während bezüglich der Ausgestaltung des Schnellspannmechanismus 3 auf die späteren Beispiele verwiesen werden soll.

Im in Figur 6 gezeigten Beispiel weist der Linearisierungsmechanismus 4 wiederum eine Kurventrommel 10 oder einen Zylinder 10 auf, in den eine Nut 8 eingebracht ist. Wiederum gleiten oder rollen in der Nut 8 Abgriffselemente 9a, 9b, 9c, die hier wiederum als Kurvenrollen 9a, 9b, 9c bzw. Wälzlager 9a, 9b, 9c ausgestaltet sind. Bezüglich der Form der Nut und der Ausgestaltung der Abgriffselemente 9a, 9b, 9c soll auf die Beschreibungen in Figur 2 verwiesen werden.

Anders als in Figur 2 sind jedoch die Achsen der Abgriffselemente 9a, 9b, 9c an den entsprechenden Lanzenaufnehmern 6a, 6b, 6c nach innen angeordnet, so dass die Abgriffselemente 9a, 9b, 9c in radialer Richtung bezüglich der Mittelachse nach innen an den entsprechenden Lanzenaufnehmern 6a, 6b, 6c angeordnet sind. Hierdurch liegen die Lanzenaufnehmer 6a, 6b, 6c an ihren dem Verbindungselement 5 zugewandten Ende nicht aneinander an und sind nicht wie in Figur 2 im Inneren der Kurventrommel 10 angeordnet, sondern sind um die Kurventrommel 10 herum angeordnet. An jenen Enden, an denen die Lanzen 2a, 2b, 2c angeordnet werden, liegen die Lanzenaufnehmer 6a, 6b, 6c jedoch aneinander an. Die Lanzenaufnehmer 6a, 6b, 6c weisen daher einen versetzten Verlauf auf, bei dem sie zunächst außerhalb der Kurventrommel 10 parallel zur Mittelachse verlaufen, dann in radialer Richtung auf die Mittelachse zu verlaufen und dann aneinander anliegend wieder parallel zur Mittelachse verlaufen.

Im in Figur 6 gezeigten Beispiel ist ein Führungssystem 11 vorgesehen, das zur Mittelachse parallele längliche Öffnungen aufweist, durch die die Lanzenaufnehmer 6a, 6b, 6c mit jenem Abschnitt hindurch verlaufen, indem sie auf die Mittelachse zu verlaufen. Die Länge der Öffnungen im Führungssystem 11 ist dabei zumindest die Amplitude des Pendelhubs der Lanzenaufnehmer 6a, 6b, 6c.

Im in Figur 6 gezeigten Beispiel kann die Antriebswelle 5 mittels eines Kugellagers bzw. Wälzlagers oder eines Gleitlagers 12 gelagert werden. Im dargestellten Fall wird die Antriebswelle 5, also das Verbindungselement 5, unterhalb der Kurventrommel 10 in einem Bauteil 13, nachfolgend auch Lageraufnahme 13 genannt, gelagert, welches fest mit dem Gehäuse verbunden ist. Die Lageraufnahme 13 kann Lineargleitlager 14b, 14c aufweisen, in welchen jeweils die Lanzenaufnehmer 9b bzw. 9c gelagert sein können, um diesen zusätzlichen Halt zu geben.

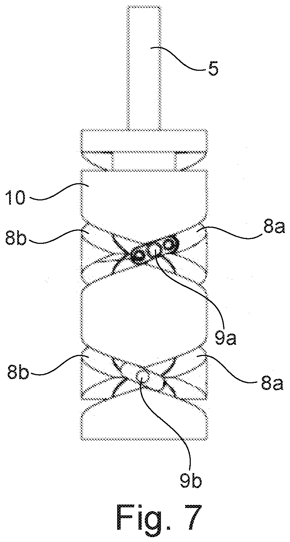

Figur 7 zeigt ein alternatives Beispiel einer Ausgestaltung einer Kurventrommel 10, wie sie in den in den Figuren 1 bis 6 gezeigten Ausgestaltungen zur Anwendung kommen kann. Die Kurventrommel 10 weist hier eine zweifach umlaufende Nut mit zwei Abschnitten 8a, 8b auf. Diese verläuft spiralförmig um die Mittelachse in der Kurventrommel 10 und geht über in eine Spiralform um die Mittelachse in entgegengesetzter Richtung. Folgt man beispielsweise dem Abschnitt 8a der Nut von jenem dem Verbindungselement nächsten Punkt, so läuft diese im gezeigten Beispiel zunächst mit konstanter Steigung nach unten, verringert um den tiefsten Punkt ihre Steigung und geht mit einer Steigung entgegengesetzten Vorzeichens in den Abschnitt 8b über, die wiederum mit konstanter Steigung nach oben bis zum höchsten Punkt verläuft, um den herum wiederum der Betrag der Steigung verringert wird.

In den Abschnitten 8a und 8b der Nut können Abgriffselemente 9a, 9b verlaufen, die an Kreuzungspunkten der Abschnitte 8a und 8b jeweils dem geradeaus liegenden nächsten Abschnitt Nut folgen. Um zu verhindern, dass die Abgriffselemente 9a, 9b an den Kreuzungspunkten in den jeweils anderen Abschnitt übergehen, können die Abgriffselemente 9a, 9b länglich ausgestaltet sein, wobei die Längsrichtung, also die Richtung größerer Erstreckung des entsprechenden Abgriffselementen 9a, 9b parallel zum Verlauf des entsprechenden Abschnitts liegt. Vorzugsweise ist die Erstreckung der Abgriffselemente 9a, 9b in der Längsrichtung größer als die Breite der Nut gemessen entlang des kreuzenden Abschnitts. Auf diese Weise wird der Übergang des Abgriffselementes 9a, 9b in den jeweils kreuzenden Abschnitt verhindert.

Auch im in Figur 7 gezeigten Beispiel können in der Spiralform auch gerade Bereiche ohne Steigung vorgesehen sein, welche eine Plateauphase, also keinen Hub des entsprechenden Lanzenaufnehmers, codieren. Durch die Anzahl der Umrundungen, die die Spirale 8a, 8b in eine Richtung ausführt, kann eine Getriebeübersetzung realisiert werden. Zwei Umrundungen, wie in Figur 7 gezeigt, führen beispielsweise zu einem Übersetzungsverhältnis von 2:1, d. h., zwei Umdrehungen des Verbindungselementes 5 führen zu einem einfachen Hub des Lanzenaufnehmers 6a, 6b, 6c in Richtung der Mittelachse.

Die Abgriffselemente 9a, 9b, 9c können hier auch dadurch länglich ausgebildet sein, dass zwei oder mehr Kurvenrollen an einem sich entlang der Längsrichtung erstreckenden Element angeordnet werden. Die Kurvenrollen eines Abgriffselements laufen also entlang der Richtung der Bahn 8a bzw. 8b hintereinander her.

Die Abgriffselemente 9a, 9b, 9c sind wiederum drehbar um eine Achse gelagert, die am Lanzenaufnehmer 6a, 6b, 6c befestigt ist. Alternativ können die Abgriffselemente 9a, 9b, 9c auch als längliche Gleitlager ausgestaltet sein.

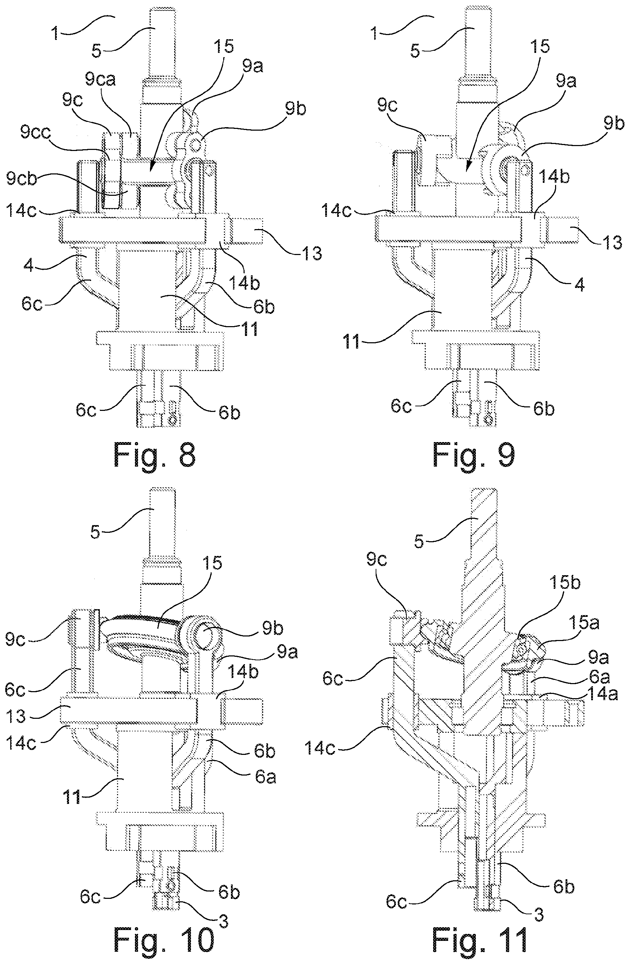

Figur 8 zeigt ein weiteres Beispiel eines erfindungsgemäßen Bohradapters, wobei wiederum nur der Linearisierungsmechanismus 4 ausgebildet ist, während bezüglich des Schnellspannmechanismus 3 auf die späteren Figuren verwiesen wird.

In den Figuren 2 bis 7 liefen die Abgriffselemente 9a, 9b, 9c in Negativ-Geometrien, wie beispielsweise Nuten. In den Figuren 8 und 9 sind Ausgestaltungen des Linearisierungsmechanismus gezeigt, in denen die Abgriffselemente 9a, 9b, 9c Positiv-Geometrien 15 umgreifen.

In den Figuren 8 und 9 sind die Abgriffselemente 9a, 9b, 9c wie in Figur 6 nach innen an den Lanzenaufnehmern 6a, 6b, 6c angeordnet. Die Lanzenaufnehmer verlaufen daher wie in Figur 6 beschrieben, so dass auf die Beschreibung dort verwiesen werden soll.

In den Figuren 8 und 9 ist jeweils ein die Mittelachse umlaufender Ring 15 vorgesehen, der im gezeigten Beispiel eben sein kann oder Plateauphasen aufweisen kann. Der Ring verläuft hier in einer gegenüber der Mittelachse nicht senkrechten Ebene. Es sei angemerkt, dass es nicht notwendig ist, dass der Ring 15 eine ebene Form hat. Durch die Form des Rings 15 können beliebige Bewegungen der Lanzenaufnehmer 6a, 6b, 6c codiert werden. Der Ring kann zum Beispiel auch im Positiv dem Negativ der Nut 8 aus Figur 2 oder 6 entsprechen.

Im in Figur 8 gezeigten Beispiel sind die Abgriffselemente 9a, 9b, 9c jeweils mit zwei Wälzlagern 9ca, 9cb ausgestaltet, von denen ein Wälzlager 9ca in Richtung der Mittelachse oberhalb des Rings 15 an diesem abrollt und das andere Wälzlager 9cb in Richtung der Mittelachse unterhalb des Rings 15 an diesem abrollt. Die Wälzlager 9ca und 9cb fassen den Ring 15 also ein. Die Wälzlager 9ca und 9cb sind an einem gemeinsamen Halteelement 9cc drehend gelagert und radial nach innen gerichtet angeordnet. Das Haltelement 9cc kann auch als Balancer bezeichnet werden. Das Haltelement 9cc ist in seiner Mitte drehbar gelagert am entsprechenden Lanzenaufnehmer 6c nach innen angeordnet. Entsprechendes gilt für die anderen Abgriffselemente 9a und 9b. Wird der Ring 15 durch das Verwendungselement 5 gedreht, so rollen die Abgriffselemente 9a, 9b, 9c mit ihren entsprechenden Rollen 9ca bzw. 9cb am Ring 15 ab und bewegen dadurch den entsprechenden Lanzenaufnehmer 6a, 6b, 6c in Richtung der Mittelachse auf und ab. Es sei angemerkt, dass stets anstelle der Wälzlager auch Gleitlager verwendet werden können.

In Figur 9 sind die Abgriffselemente 9a, 9b, 9c als einstückige Elemente ausgestaltet, die jeweils eine Nut aufweisen, in welcher der Ring 15 liegt. Der Ring 15 gleitet also in der Nut der Abgriffselemente 9a, 9b, 9c. Die Abgriffselemente 9a, 9b, 9c sind an den entsprechenden Lanzenaufnehmern 6a, 6b, 6c drehbar gelagert, wobei die Drehachse vom Lanzenaufnehmer 6a, 6b, 6c gesehen radial nach innen gerichtet ist und senkrecht zur Mittelachse steht. Im gezeigten Beispiel haben die Abgriffselemente 9a, 9b, 9c um die entsprechende Drehachse einen kreisförmigen Umfang, was jedoch nicht notwendig ist. In Figur 9 können die Abgriffselemente 9a, 9b, 9c auch als Klammern bezeichnet werden.

Die Drehbarkeit der Abgriffselemente 9a, 9b, 9c um die an dem entsprechenden Lanzenaufnehmer 6a, 6b, 6c angeordnete Drehachse ermöglicht eine Verkippung des entsprechenden Abgriffselementes 9a, 9b, 9c entsprechend dem Verlauf der Bahn bzw. des Ringes 15.

Die Figuren 10 und 11 zeigen eine weitere Ausgestaltung eines erfindungsgemäßen Bohradapters 1, wobei wiederum der Linearisierungsmechanismus 4 im Detail gezeigt ist, während bezüglich des Schnellspannmechanismus auf die späteren Figuren verwiesen werden soll. Zeigt Figur 10 diese Ausgestaltung perspektivisch und Figur 11 im Schnitt.

In den Figuren 10 und 11 weist der Ring 15 zwei Komponenten auf, nämlich zum einen einen äußeren Ring 15a sowie einen inneren Ring 15b, die in einer gemeinsamen Ebene verlaufen, die gegenüber der Mittelachse in einem nicht senkrechten Winkel steht. Dabei ist der äußere Ring 15a gegenüber dem inneren Ring 15b gelagert, beispielswiese über Kugellager, so dass der innere Ring 15b gegenüber dem äußeren Ring 15a drehbar ist. Der innere Ring 15b ist fest gegenüber dem Verbindungselement 5 und wird durch dieses zur Drehung angetrieben. Die Ringe 15a und 15b bilden also ein Lager, beispielsweise ein Wälzlager oder ein Gleitlager, das schräg an der Antriebswelle 5, d. h. dem Verbindungselement 5, angebracht ist. Außen am äußeren Ring 15a greifen hier die Abgriffselemente 9a, 9b, 9c an, die jeweils mit dem äußeren Ring 15a formschlüssig in Kontakt stehen. Die Abgriffselemente 9a, 9b, 9c weisen hierzu jeweils eine Nut auf, in welcher der Ring 15a liegt. Jene die Nut aufweisenden Abgriffselemente sind drehbar gelagert um eine senkrecht zur Mittelachse stehende Achse. Das Lager ist hierbei am Ende des jeweiligen Lanzenaufnehmers 6a, 6b, 6c angeordnet.

Die Drehung des Verbindungselementes 5 wird hier also nicht auf den äußeren Ring 15a übertragen, jedoch die durch die Drehung erzeugte Auf- und Abbewegung schon. Diese wird dann über die drehbar mit den Lanzenaufnehmern 6a, 6b, 6c gelagerten Abgriffselemente 9a, 9b, 9c in einer Auf- und Abbewegung der Lanzenaufnehmer 6a, 6b, 6c überführt.

Die Lanzenaufnehmer 6a, 6b, 6c sind hier im oberen Bereich wieder außen angeordnet und werden nach unten hin zusammengeführt. Diesbezüglich soll auf die Beschreibung zur Figur 6 verwiesen werden.

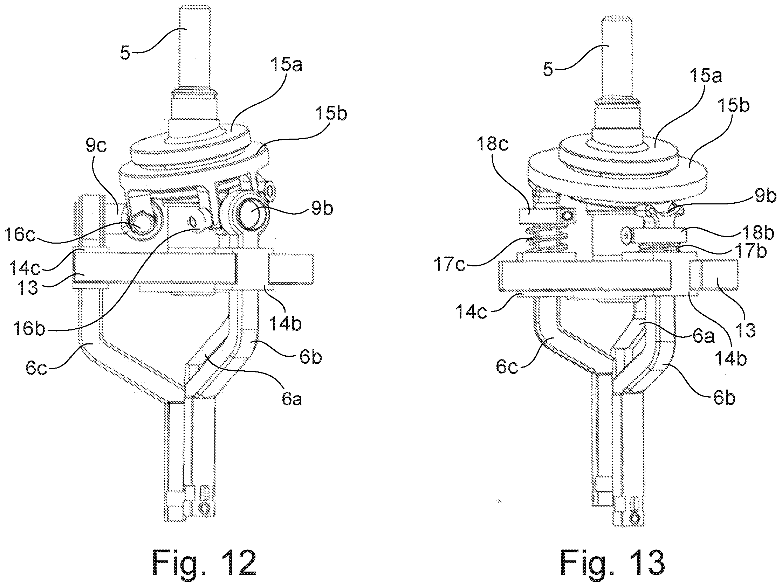

Figur 12 zeigt eine Variante der in den Figuren 10 und 11 gezeigten Ausgestaltung. Es soll von der Beschreibung zu den Figuren 10 und 11 ausgegangen werden.

Im in Figur 12 gezeigten Beispiel sind wiederum zwei gegeneinander gelagerte Ringe 15a und 15b vorgesehen, wobei wiederum einer der Ringe 15a am Verbindungselement 5 fest angeordnet ist und wobei die Abgriffselemente 9a, 9b, 9c am anderen Ring 15b angreifen. Der Ring 15a ist dabei auf der den Lanzen abgewandten Seite des Rings 15b angeordnet.

Die Abgriffselemente 9a, 9b, 9c weisen hier jeweils über ein scharnierartiges Gelenk 16a, 16b, 16c verbundene Schenkel auf, von denen ein Schenkel an dem entsprechenden Lanzenaufnehmer 6a, 6b, 6c um eine Achse senkrecht zur Mittelachse drehbar gelagert ist, und wobei der andere Schenkel fest mit dem Ring 15a verbunden ist. Die Drehachsen der scharnierartigen Gelenke 16a, 16b, 16c liegen tangential zu einem Kreis, der parallel verläuft zum Ring 15a. Die Ringe 15a und 15b können z. B. über Axialnadellager oder Axialzylinderrollenlager gegeneinander gelagert sein.

Im in Figur 12 gezeigten Beispiel wird die Auf- und Abbewegung, d. h. die Pendelbewegung am Ring 15b nicht nur radial nach außen, sondern auch nach unten abgegriffen. Die scharnierartigen Gelenke 16a, 16b, 16c fangen die Verkippung der Scheibe auf und lassen eine Linearbewegung jener am entsprechenden Lanzenaufnehmer 6a, 6b, 6c gelagerten Schenkel der Abgriffselemente 9a, 9b, 9c zu. Auch Kugelgelenke oder andere Konfigurationen, die Rotation um zwei unterschiedliche Achsen ermöglichen, sind möglich.

Figur 13 zeigt eine weitere Variante der in den Figuren 10 bis 12 gezeigten Ausgestaltung. Wiederum sind zwei gegeneinander gelagerte Ringe 15a und 15b vorgesehen, von denen der Ring 15a mit dem Verbindungselement 5 fest verbunden ist. Die Abgriffselemente 9a, 9b, 9c sind im in Figur 13 gezeigten Beispiel als Nuten am Ende der Lanzenaufnehmer 6a, 6b, 6c ausgebildet, in die jeweils ein Vorsprung, der am Ring 15b fest verbunden ist, einliegt. Bei Drehung des Verbindungselementes 5 führt der entsprechende Vorsprung eine Auf- und Abbewegung aus, wobei in der Abbewegung, d. h. der Bewegung in Richtung der Bohrlanzen, der Ring 15b über den Vorsprung den entsprechenden Lanzenaufnehmer 6a, 6b, 6c nach unten drückt. Um die Lanzenaufnehmer 6a, 6b, 6c mit dem Ring 15b in Kontakt zu halten, wenn dieser sich nach oben, also in Richtung des Verbindungselementes 5, bewegt, sind Federn 17a, 17b, 17c vorgesehen, deren Federkraft den entsprechenden Lanzenaufnehmer 6a, 6b, 6c nach oben drückt. Zur Übertragung der Federkraft auf den entsprechenden Lanzenaufnehmer 6a, 6b, 6c ist an jedem der Lanzenaufnehmer 6a, 6b, 6c jeweils ein fixiertes Element 18a, 18b, 18c angeordnet, das auch in den entsprechenden Lanzenaufnehmer integriert sein kann. Die Feder ist dann zwischen dem Führungselement 13 oder dem Gleitlager 14a, 14b, 14c und dem entsprechenden fixieren Element 18a, 18b, 18c angeordnet. Da die Lanzenaufnehmer 6a, 6b, 6c am äußeren Rand des Rings 15b angreifen, werden sie nach unten hin durch einen abgeknickten Verlauf zueinander geführt, wie diesbezüglich Figur 6 beschrieben wurde.

Die Vorsprünge der Abgriffselemente 9a, 9b, 9c, die am Ring 15b angeordnet sind, können beispielsweise eine Halbkugel- oder Zylinderform haben. Sie können in der Nut am Ende des entsprechenden Lanzenaufnehmers 6a, 6b, 6c verdrehbar und/oder verschiebbar sein.

Es sei angemerkt, dass grundsätzlich in allen oben beschriebenen Ausgestaltungen die Lanzenaufnehmer mittels Federn rückgestellt werden können. In Figur 2 und 6 wäre es dann zum Beispiel ausreichend, wenn nur die obere Fläche der Nut 8 vorgesehen wäre, wobei die Federn jeweils das entsprechende Abgriffselement gegen diese Fläche drücken.

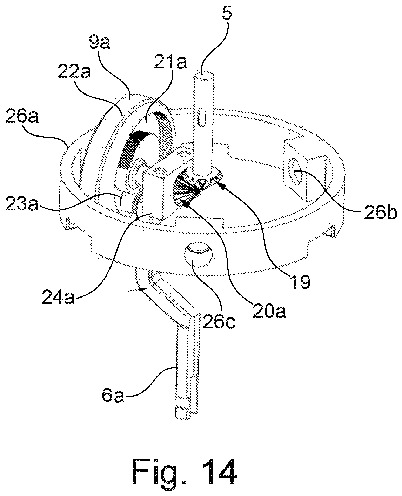

Figur 14 zeigt ein weiteres Beispiel eines Teils eines Linearisierungsmechanismus 4 eines erfindungsgemäßen Bohradapters 1. In diesem Beispiel ist am der Antriebseinheit abgewandten Ende des Verbindungselementes 5 ein Kegelrad 19 vorgesehen, in das für jedes der Abgriffselemente 9a, 9b, 9c ein Kegelrad 20a eingreift, dessen Drehachse senkrecht zur Mittelachse steht. In Figur 14 ist nur ein Lanzenaufnehmer 6a mit einem Abgriffselement 9a gezeigt. Weitere Lanzenaufnehmer 6b, 6c mit weiteren Abgriffselementen 9b, 9c sind wie für den Lanzenaufnehmer 6a gezeigt ausgestaltet und an den Bohrungen 26b und 26c angeordnet, hier jedoch zur Erhöhung der Übersichtlichkeit nicht gezeigt.

Das Kegelrad 20a ist hier in einem Element 24a gelagert, das fest am Gehäuse angeordnet ist.

Die Kurvenscheiben 22a weisen eine Nut 21a in ihrer den Mittelachsen zugewandten Oberfläche auf. Diese Oberfläche steht parallel zur Mittelachse. Die Abgriffsrolle 23a läuft in der Nut 21a. Durch geeignete Wahl der Form der Nut 21a kann jede beliebige Auf- und Abbewegung des Lanzenaufnehmers 6a realisiert werden.

Sollen drei oder mehr gleiche Kurvenscheiben 22a vorgesehen werden, so ist es vorteilhaft, wenn diese jeweils um einen definierten Winkel verdreht auf der Achse angebracht werden. Dies kann beispielsweise dadurch gelöst werden, dass die Kurvenscheibe 22a jeweils über eine Passfedernut auf der Welle des entsprechenden Kegelrads 20a angeordnet wird. Auf diese Weise kann die Bewegung der Lanzenaufnehmer 6a, 6b, 6c für jeden der Lanzenaufnehmer 6a, 6b, 6c unabhängig realisiert werden. Es können auch unterschiedliche Bahnformen in den unterschiedlichen Kurvenscheiben 22a vorgesehen werden. Die Bewegungen der Lanzenaufnehmer sind hier nicht vollkommen unabhängig, da sie durch das gleiche Kegelrad angetrieben werden. Durch die unterschiedlich positionierten Passfedernuten kann aber die zyklusabhängige Position im Verhältnis zu den anderen Lanzenaufnehmern definiert werden (die Einstellung ist das Pendant zu den Winkeln zwischen den Abgriffselementen (vgl. Fig. 3 vs. Fig. 4)).

Die Abgriffsrolle 23a überträgt die Bewegung der Kurvenscheibe 22a auf den entsprechenden Lanzenaufnehmer 6a. Anstelle einer Kurvenrolle kann hier auch ein Wälz- oder Gleitlager zum Einsatz kommen. Die Abgriffsrolle 23a befindet sich hier in der Bahn in 21a in der Kurvenscheibe 22a. Die Lanzenaufnehmer 6a können wie in den anderen Beispielen in einem Führungssystem geführt werden, so dass sie sich nur auf und ab bewegen, jedoch nicht mitdrehen. Bei Drehung der Kurvenscheibe 22a ändert sich der Abstand der Bahn und somit der Abgriffsrolle 23a zum Drehzentrum, d. h. der Drehachse des Kegelrads 20a, wodurch Hub erzeugt wird. Alternativ zu einer Kurvenscheibe 22a mit Nut 21a kann die Bewegung auch außen (Nocken) oder innen abgegriffen werden und der Lanzenaufnehmer 6a dann mit einer Feder zurückgestellt werden.

Zwischen den Kegelrädern 19 und 20a kann eine Übersetzung vorgesehen werden, beispielsweise, um die Drehzahl zu erhöhen oder zu vermindern. Die Kegelräder 20a sind auf Wellen bzw. Achsen angeordnet, welche auf das Drehzentrum der Antriebsachse bzw. die Mittelachse ausgerichtet sind. Wie bereits vorstehend erwähnt, können sie auch hier gleichmäßig oder ungleichmäßig über den Umfang um die Mittelachse verteilt sein. Die Lagerung der Wellen am Element 24a und am Gehäusering in der Öffnung 26a erhöht die Stabilität. Die Wellen sind verdrehsicher und axial gesichert mit den Kurvenscheiben 22a verbunden, z. B. durch eine Passfeder, Keilwellenverbindung, über Kraftschluss oder Ähnliches.

Die Kurvenscheiben 22a weisen eine zweidimensionale Bahn, hier eine Nut 21a, auf, durch welche eine definierte Auf- und Abbewegung der Lanzenaufnehmer 6a übertragen werden kann. Die Form der Bahn 21a kann frei gewählt werden, so dass die Bewegung der Lanzenaufnehmer 6a frei programmierbar ist. Zum Beispiel können auch Plateauphasen ohne Hubbödenänderungen über einen gewissen Zyklusabschnitt vorgesehen werden.

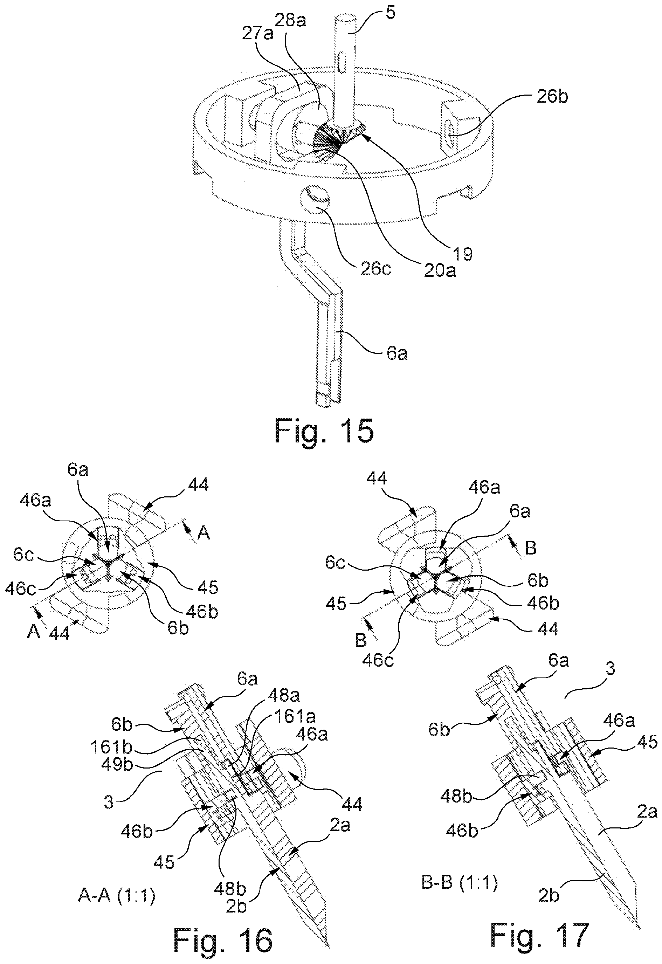

Alternativ können anstelle von Kurvenscheiben 22a mit Abgriffrollen 23a auch andere Mechanismen eingesetzt werden, um bei Drehung der Achse eine Auf- und Abbewegung der Lanzenaufnehmer 6a, 6b, 6c zu erzeugen. Diese können das Prinzip der Exzentrizität verwenden. Dabei wird durch das Kegelrad 20a ein exzentrischer Körper um die Rotationsachse des Kegelrads 20a gedreht. Dies ist beispielhaft in Figur 15 gezeigt. Figur 15 zeigt dabei der Übersichtlichkeit halber wieder nur einige Komponenten und insbesondere nur den Mechanismus eines Lanzenaufnehmers 6a, wobei jedoch für weitere Lanzenaufnehmer 6b, 6c entsprechende Mechanismen vorgesehen sind, die wiederum an den Öffnungen 26b und 26c angeordnet sind.

In Figur 15 treibt das Kegelrad 20a einen über eine Welle mit diesem verbundenen Exzenter 28a an, der in einer länglichen Öffnung 27a liegt. Wird der Exzenter 28a um die Drehachse des Kegelrads 20a gedreht, so bewegt er das Abgriffselement 27a auf und ab und bewegt damit den Lanzenaufnehmer 6a auf und ab.

Im Normalfall wird hierdurch eine Bewegung des Lanzenaufnehmers 6a erzeugt, die trigonometrisch ist, also einem Sinus oder Cosinus folgt, wobei der Hub dann keine Plateauphasen aufweist. Die Exzenter 28a sind hier mit der Welle des Kegelrads 20a verdrehsicher verbunden, beispielsweise mittels einer Passfeder-Passfedernut-Verbindung, wobei die Nuten in den Exzentrizitäten über den Umfang verteilt wie im vorstehend beschriebenen Fall sein können. Den Exzentrizitäten 28a ist jeweils auf Seiten der Lanzenaufnehmer 6a eine Nutgeometrie 27a zugeordnet, die die Exzentrizität zu den Seiten ausgleicht. Die Lanzenaufnehmer 6a werden also nicht seitlich verschoben. Eine Bewegung in Richtung der Mittelachse (Hub) ist jedoch möglich. Bei Bewegung des Exzenters 28a nach oben oder unten wird der Lanzenaufnehmer nach oben oder unten gedrückt. Alternativ zu der in Figur 15 gezeigten länglichen Nut-Geometrie kann auch eine kreisförmige Geometrie zum Einsatz kommen, wobei der Exzenter 28a von ihr vollständig eingefasst sein kann. Der Exzenter 28a kann also verdrehbar in der Nut-Geometrie 27a gelagert sein. In diesem Fall ist jedoch eine im Lanzenaufnehmer 6a gelenkig gelagerte Stange vorteilhaft, welche an der Nut-Geometrie 28a angebracht oder mit dieser verbunden sein kann, beispielsweise ein Kurbeltrieb.

Alternativ zu der beschriebenen Anordnung der Achsen, bei welcher mehrere Achsen durch die Antriebswelle angetrieben werden, und bei der die Anzahl der Achsen gleich der Anzahl der Bohrraspeln ist, kann auch eine andere Konfiguration gewählt werden. Dabei können weniger oder auch nur eine Achse vorgesehen werden, die dann mehrere der Exzentrizitäten aufweisen können. Es können also mehrere Exzenter 28a auf einer gemeinsamen Welle angeordnet sein. Diese können zueinander verdreht sein, gleichmäßig oder ungleichmäßig. Die Form der Lanzenaufnehmer 6a und die Position der Linearführungen 14a können dann entsprechend angepasst sein. Es ist darüber hinaus auch möglich, die Exzentrizitäten direkt auf der Antriebswelle 5 anzuordnen, wodurch dann eine Bewegung der Lanzenaufnehmer 6a senkrecht zur Mittelachse erzeugt werden kann.

Im Folgenden sollen nun einige Beispiele für Schnellspannmechanismen 3 aufgezeigt werden. Alle im Folgenden gezeigten Schnellspannmechanismen 3 können zusammen mit allen vorstehend beschriebenen Ausgestaltungen der Linearisierungsmechanismen 4 realisiert werden.

Figur 16 und 17 zeigen beispielhaft eine mögliche Ausgestaltung des Schnellspannmechanismus 3, mit dem die Bohrlanzen 2a, 2b, 2c an den Lanzenaufnehmern 6a, 6b, 6c befestigbar sind. Dabei zeigt Figur 16 den Schnellspannmechanismus in einem geöffneten Zustand, in dem die Lanzen aus den Lanzenaufnehmern 6a, 6b, 6c entnehmbar sind, während Figur 17 einen geschlossenen Zustand zeigt, in dem die Lanzen 2a, 2b, 2c fest in den Lanzenaufnehmer 6a, 6b, 6c befestigt sind. Es zeigt dabei jeweils die obere Teilfigur eine Draufsicht und die untere Teilfigur einen Schnitt.

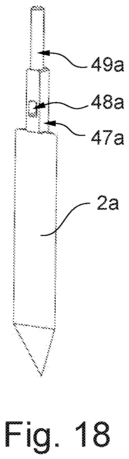

Figur 18 zeigt beispielhaft eine mögliche Ausgestaltung einer Bohrlanze 2a, die in den Figuren 16 und 17 gezeigten Schnellspannmechanismus einsetzbar ist. Jeder der Lanzenaufnehmer 6a, 6b, 6c weist jeweils einen Aufnahmekanal 161a, 161b auf, in den ein Einführelement der entsprechenden Bohrlanze 2a, 2b, 2c einführbar ist. Wie in Figur 18 gezeigt, können die Bohrlanzen 2a ein Einführelement aufweisen, das hier zum einen einen Abschnitt 47a mit quadratischem Querschnitt sowie einen Abschnitt 49a mit rundem Querschnitt aufweisen. Entsprechend weisen die Einführkanäle 161a, 161b entsprechende Abschnitte mit entsprechenden Querschnitten auf. In den Figuren 16 und 17 ist zu erkennen, dass die Bohrlanzen mit den Einführelementen in die entsprechenden Aufnahmekanäle 161a, 161b eingeführt sind.

Es sei darauf hingewiesen, dass der Aufnahmekanal nicht um seine zur Mittelachse parallele Richtung vollständig geschlossen sein muss. Es sollen im Allgemeinen auch Strukturen als Aufnahmekanal angesehen werden, die nur auf einem Teil des Umfangs um jene Achse entlang der die Lanze in den Lanzenaufnehmer 6 eingeführt wird, vorliegen.

Der Schnellspannmechanismus weist für jede Bohrlanze 6a, 6b, 6c jeweils einen Verschlussschieber 46a, 46b, 46c auf, der in radialer Richtung verschiebbar ist. Die Verschlussschieber 46a, 46b, 46c weisen jeweils ein Verschlusselement auf, das in radialer Richtung koaxial zu einer Bohrung 48a im Einführelement 47a der Bohrlanzen 2a, 2b, 2c eingebracht ist. Durch Verschieben des Verschlussschiebers 46a, 46b, 46c in radialer Richtung kann das Verschlusselement aus der jeweiligen Bohrung 48a heraus oder in diese hinein bewegt werden.

Der Schnellspannmechanismus 3 weist außerdem eine äußere Hülse 45 auf, welche die Bohrlanzen 2a, 2b, 2c und die Verschlussschieber 46a, 46b, 46c umläuft. Die äußere Hülse 45 hat eine innere Oberfläche, die in einer der Zahl der Lanzenaufnehmer 6a, 6b, 6c entsprechenden Anzahl von Bereichen einen ersten Abstand von der Mittelachse hat und in einer der Zahl der Bohrlanzen 2a, 2b, 3c entsprechenden Anzahl von Bereichen einen zweiten Abstand von der Mittelachse hat. Dabei sind in Richtung entlang des Umfangs jeweils abwechselnd ein Bereich mit dem ersten Abstand und ein Bereich mit dem zweiten Abstand vorgesehen. In dem in Figuren 16 und 17 gezeigten Beispiel gibt es also drei Bereiche mit dem ersten Abstand und drei Bereiche mit dem zweiten Abstand. Dies ist in der Draufsicht der Figuren 16 und 17 deutlich zu erkennen.

Der erste Abstand ist größer als der zweite Abstand. Dabei ist der erste Abstand so bemessen, dass, wenn die Verschlussschieber 46a, 46b, 46c in diesen Bereichen liegen, sich die Verschlusselemente nicht in der jeweiligen Öffnung 48a der Bohrlanze 2a befinden. Die zweiten Abstände sind hingegen so bemessen, dass, wenn sich die Verschlussschieber 46a, 46b, 46c in den Bereichen dieser Abstände befinden, die Verschlusselemente vollständig in die jeweilige Öffnung 48a eingeführt sind.

Sind die Verschlusselemente in die jeweilige Öffnung 48a der entsprechenden Bohrlanze 2a eingeführt, so wird die Bohrlanze 2a im Aufnahmekanal 161a gehalten. Ist das Verschlusselement hingegen nicht in der Öffnung 48a, so ist die Bohrlanze 2a frei und kann aus dem Aufnahmekanal 161a entfernt werden. Entsprechendes gilt für die anderen Bohrlanzen 2b, 2c.

Figur 16 zeigt diese Ausgestaltung des Schnellspannmechanismus 3 im geöffneten Zustand. In der Draufsicht ist zu erkennen, dass die Verschlussschieber 46a, 46b und 46c in den Bereichen des ersten, größeren Abstands liegen. Im Schnitt ist zu erkennen, dass die Verschlusselemente der Verschlussschieber 46a, 46b nicht in der entsprechenden Öffnung 48a eingeführt sind. In Figur 17 befindet sich der Schnellspannmechanismus 3 im geschlossenen Zustand. Im Schnitt ist zu erkennen, dass die Verschlusselemente der Verschlussschieber 46a, 46b in die entsprechende Öffnung 48a eingeführt sind.

In den Figuren 16 und 17 weist die Hülse 45 auf ihrer äußeren Oberfläche, die im wesentlichen kreisförmig ist, optionale Knöpfe 44 auf, mit denen die Hülse 45 um die Mittelachse drehbar ist. Durch Drehen der Hülse 45 um die Mittelachse kann der Schnellspannmechanismus 3 vom in Figur 16 gezeigten geöffneten Zustand in den in Figur 17 gezeigten geschlossenen Zustand überführt werden und andersherum.

In den Figuren 16 und 17 haben also die Lanzenaufnehmer 6a, 6b, 6c jeweils eine Aufnahme, in welche die entsprechende Bohrraspel 2a, 2b, 2c formschlüssig eingeschoben werden kann. Dabei hat die Bohrraspel 2a, 2b, 2c vorzugsweise zumindest ein Element 47a, welches nicht rotierbar ist und als positiv exakt formschlüssig in das Negativ des Lanzenaufnehmers 6a, 6b, 6c passt. Im dargestellten Beispiel hat die Bohrlanze 2a, 2b, 2c auch noch einen zylindrischen Bereich 49a, welcher im dazu passenden Negativ des Lanzenaufnehmers 6a zentriert wird und zusätzlichen Halt gibt. Hier werden die Kräfte in Einschubrichtung beim Bohren über die Stirnfläche dieses zylindrischen Stücks übertragen. Auf diese Weise sind alle Bewegungsfreiheitsgrade der Bohrlanzen 2a, 2b, 2c gegenüber den Lanzenaufnehmern 6a, 6b, 6c blockiert, nämlich durch die Aufnahmen und/oder die anderen Bohrlanzen 2a, 2b, 2c, mit Ausnahme der Bewegungsmöglichkeit entgegen der Einschubrichtung. Durch Einschieben der Verschlusselemente in die Aussparungen 48a kann dieser letzte Freiheitsgrad fixiert und gelöst werden, so dass die Bewegung entgegen der Einschubrichtung verhindert wird. Das Vorsehen der kontrollierten Hülse 45, die wie oben beschrieben ausgestaltet ist, ermöglicht ein schnelles Fixieren und Lösen.

Der erfindungsgemäße Bohradapter kann ein Gehäuse aufweisen, das in Figur 1 zu erkennen ist, und in den Figuren 16 und 17 zur Erhöhung der Übersichtlichkeit weggelassen wurde. Dieses Gehäuse kann eine radial teilweise umlaufende Nut aufweisen, durch die die Knöpfe oder Griffe 44 sich hindurch erstrecken, wie dies in Figur 1 zu erkennen ist.

Der Prozess des Befestigens der Bohrlanzen kann also nach dem folgenden Ablauf erfolgen. Es werden zunächst die Bohrlanzen 2a, 2b, 2c eingeschoben, bis sie vollständig in den Aufnahmen 161a, 161b, 161c sitzen. Anschließend wird der Griff 44, und damit die Hülse 45, um die Mittelachse verdreht, wodurch die Verschlussschieber 46a, 46b, 46c mit den Verschlusselementen jeweils in die entsprechende Bohrung 48a im Einführelement der entsprechenden Bohrlanze 2a, 2b, 2c gleiten. Auf diese Weise können alle Bohrlanzen 2a, 2b, 2c gleichzeitig fixiert werden.

Die Aufnahmekanäle 161a, 161b, 161c weisen jeweils ebenfalls eine Durchgangsöffnung auf, die koaxial zur Durchgangsöffnung 48a der entsprechenden Bohrlanze liegt, wenn die entsprechende Bohrlanze 2a, 2b, 2c vollständig in den entsprechenden Lanzenaufnehmer 6a, 6b, 6c eingeführt ist. Die Verschlussschieber 46a, 46b, 46c und die Verschlusselemente können so bemessen sein, dass die Verschlusselemente im geöffneten Zustand nicht in der Öffnung 48a der entsprechenden Bohrlanze 2a sitzen, jedoch in der hierzu koaxialen Öffnung im Lanzenaufnehmer 6a. Bei bestimmungsgemäß eingeführter Bohrlanze 2a kann die Durchgangsrichtung der Öffnung 48a und der dazu koaxialen Öffnung in den Lanzenaufnehmer 6a radial zur Mittelachse verlaufen.

Sind alle Bohrlanzen 2a, 2b, 2c fixiert, so bewegen sie sich nun wie eine Einheit mit dem entsprechenden Lanzenaufnehmer 6a, 6b, 6c, der sich durch die Bewegungslinearisierung mittels des Linearisierungsmechanismus 4 auf und ab bewegt. Zum Lösen muss nur der Knopf 44 wieder um die Mittelachse verschoben werden.

Vorzugsweise ist zwischen den Verschlussschiebern 46a, 46b, 46c und der Oberfläche des entsprechenden Lanzenaufnehmers 6a, 6b, 6c eine Rückstelleinheit wie beispielsweise eine Druckfeder vorgesehen, die dafür sorgt, dass sich der Verschlussschieber 46a, 46b, 46c selbständig aus der Öffnung 48a der Bohrlanze 2a entfernt, sobald dies durch genügend Platz mit dem ersten Abstand ermöglicht wird.

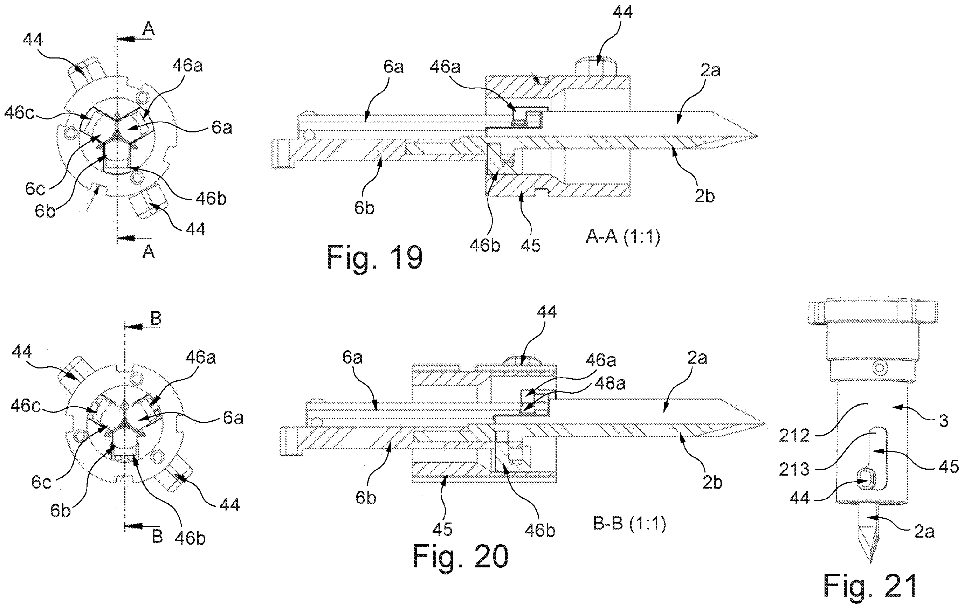

Die Figuren 19, 20 und 21 zeigen eine alternative vorteilhafte Ausgestaltung eines Schnellspannmechanismus, wie er im erfindungsgemäßen Bohradapter 1 zum Einsatz kommen kann.

Dabei zeigt Figur 19 den Schnellspannmechanismus 3 im geschlossenen Zustand, in dem die Bohrlanzen 2a, 2b in den Lanzenaufnehmern 6a, 6b fixiert sind und Figur 20 zeigt den Schnellspannmechanismus 3 im geöffneten Zustand.

In dem hier gezeigten Beispiel weist der Schnellspannmechanismus 3 wieder für jede der Lanzenaufnehmer 6a, 6b, 6c einen Verschlussschieber 46a, 46b, 46c auf, der jeweils ein Verschlusselement hat. Wiederum weist jeder Lanzenaufnehmer 6a, 6b, 6c einen Aufnahmekanal auf, sowie eine Durchgangsöffnung auf Höhe des Aufnahmekanals, die sich radial nach außen erstreckt. Die Bohrlanzen können wie in Figur 18 gezeigt ausgestaltet sein. Die dort gezeigte Öffnung 48a ist bei bestimmungsgemäßer Position der Bohrlanzen 2a, 2b, 2c im entsprechenden Aufnahmekanal des entsprechenden Lanzenaufnehmers 6a, 6b, 6c koaxial zur genannten Durchgangsöffnung im Lanzenaufnehmer 6a, 6b, 6c. Durch Verschieben des Verschlussschiebers 46a, 46b, 46c kann also das Verschlusselement durch die Öffnung im Lanzenaufnehmer 6a, 6b, 6c in die Öffnung 48a der Bohrlanze 2a, 2b, 2c eingeführt werden.

Die Aufnahmekanäle der Lanzenaufnehmer 6a, 6b, 6c können wie zu den Figuren 16 und 17 beschrieben ausgestaltet sein.

Auch in den Figuren 19 und 20 ist eine Hülse 45 vorgesehen, die wiederum eine innere Oberfläche hat, die hier jedoch zwei Bereiche mit unterschiedlichen Abständen von der Mittelachse aufweist. Dabei hat ein erster Bereich der inneren Oberfläche der Hülse 45, der den Bohrlanzen 2a, 2b, 2c zugewandt ist, einen ersten Abstand von der Mittelachse, und ein den Bohrlanzen abgewandter Bereich einen zweiten Abstand von der Mittelachse. Wiederum ist der erste Abstand größer und so bemessen, dass wenn, wie in Figur 20 gezeigt, die Verschlussschieber 46a, 46b, 46c in diesem Bereich vorliegen, sich die Einführelemente der Verschlussschieber 46a, 46b, 46c in der Durchgangsöffnung zum Aufnahmekanal der Lanzenaufnehmer 6a, 6b, 6c befinden, jedoch nicht in der Durchgangsöffnung 48a im Einführelement der Bohrlanzen 2a, 2b, 2c. Im in Figur 20 gezeigten Beispiel sind die Bohrlanzen daher nicht in Richtung der Mittelachse fixiert.

Hingegen ist der zweite Abstand kleiner als der erste Abstand und so bemessen, dass wenn sich die Verschlussschieber 46a, 46b, 46c in diesem Bereich der inneren Oberfläche der Hülse 45 befinden, die Verschlusselemente in die Durchgangsöffnung 48a der Bohrlanzen 2a, 2b, 2c eingeführt sind. Die Bohrlanzen sind hierdurch fixiert.

Die Hülse 45 weist hier wiederum einen Griff oder Knopf 44 auf, mit dem die Hülse von außerhalb eines Gehäuses 212 des Schnellspannmechanismus oder des Bohradapters beweglich ist. In dem Gehäuse 212 ist hierzu eine Nut 213 eingebracht, durch die hindurch sich der Knopf 44 erstreckt. Die Nut 213 verläuft zumindest teilweise parallel zur Mittelachse, so dass sich durch Verschieben des Knopfes 44 in der Nut 213 die Hülse 45 von der geöffneten zur geschlossenen Position und andersrum bewegen lässt. Die Nut 213 kann an ihrem den Lanzen zugewandten Ende abgeknickt sein, wodurch es möglich wird, den Knopf 44 und damit die Hülse 45 in der geschlossenen Stellung zu arretieren. Dies ist in Figur 21 gezeigt.

Der Bereich der inneren Oberfläche der Hülse 45, der den zweiten Abstand zur Mittelachse hat, sollte in Richtung der Mittelachse zumindest so breit sein, wie die Amplitude der Hubbewegung während des Bohrprozesses. Hierdurch wird gewährleistet, dass sich der Verschlussschieber 46a, 46b, 46c in allen Positionen während des Bohrens im geschlossenen Zustand befindet.

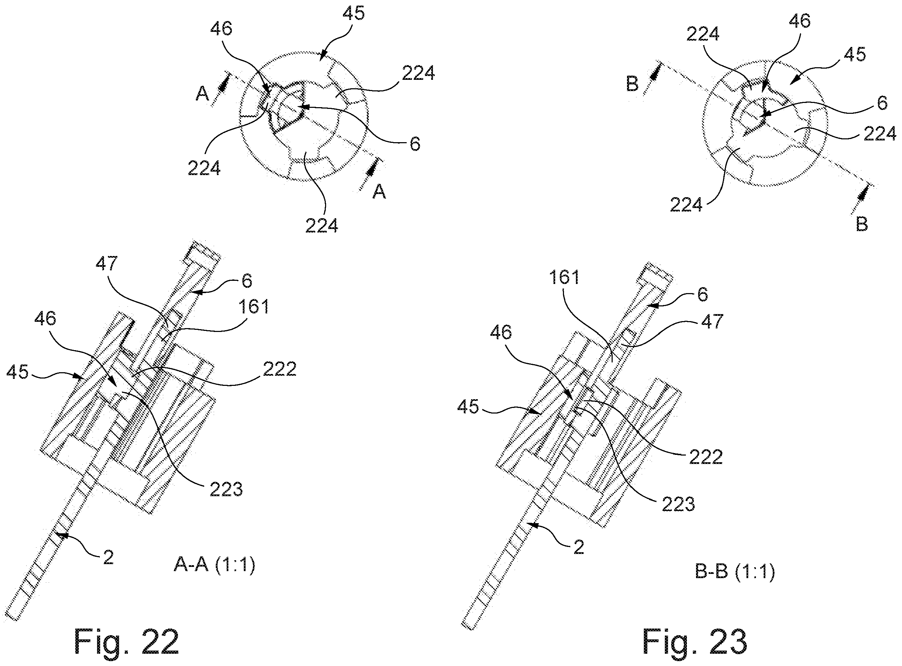

Die Figuren 22 und 23 zeigen eine weitere mögliche Ausgestaltung des Schnellspannmechanismus 3 zur Verwendung im erfindungsgemäßen Bohradapter 1. Es zeigt Figur 22 den geschlossenen Zustand und Figur 23 den geöffneten Zustand. Der Übersichtlichkeit halber ist hier nur eine Bohrlanze 2 mit einem Verschlussschieber 46 eingezeichnet. Die anderen Bohrlanzen 2a, 2b, 2c sind an ihren entsprechenden Winkelpositionen mit entsprechenden Verschlussschiebern 46a, 46b, 46c angeordnet.

Im hier gezeigten Beispiel weist die Bohrlanze 2 in ihrem Einführbereich 47 eine Aussparung 222 auf. Darüberhinaus weist der Lanzenaufnehmer 6 im Bereich seines Aufnahmekanals 161 eine Aussparung 223 auf, die, wenn die Lanze 2 bestimmungsgemäß in den Lanzenaufnehmer 6 eingeführt ist, in Richtung der Mittelachse auf gleicher Höhe liegt wie die Aussparung 222 der Bohrlanze.

Die Aussparung 222 der Bohrlanze 2 wird dadurch gebildet, dass auf einem kreiszylinderförmigen Umfang des Einführelementes 47 in Richtung entlang der Mittelachse vor und hinter der Aussparung 222 Verbreiterungen hergestellt werden, in denen sich das Einführelement von der Zylinderachse der besagten Zylinderform radial weiter nach außen erstreckt als im Bereich der Ausnehmung. Diese Vorsprünge sind auf einen Winkelbereich beschränkt. Der Lanzenaufnehmer kann in seinem Aufnahmekanal 161 in seiner inneren Oberfläche eine parallel zur Achse des Aufnahmekanals verlaufenden Nut aufweisen, in der dieser Bereich vergrößerter Dicke des Einführelementes 47 verschoben wird. Der Verschlussschieber 46 kann nun um die Mittelachse herum verschoben werden, zwischen einer Position, in der er mit einem radial nach innen gerichteten Vorsprung in die Ausnehmung 222 der Bohrlanze und die Ausnehmung 223 des Lanzenaufnehmers 6 eingreift und einer Position, in der er nicht in die Ausnehmung 222 der Lanze 2 eingreift. Hierzu wird der Verschlussschieber in eine Winkelposition um die Mittelachse verschoben, in der der Bereich erhöhter Dicke des Einführelementes 47 nicht vorliegt. Der Bereich erhöhter Dicke kann sich daher also in Richtung der Mittelachse an dem Verschlussschieber 46 vorbeibewegen, so dass die Lanze aus dem Lanzenaufnehmer 6 entfernbar ist. Zum Verschließen kann der Verschlussschieber 46 wieder in die Öffnung 222 und 223 eingeschoben werden.

Im gezeigten Beispiel sind die Verschlussschieber 46 jeweils in einer Nut 224 in einer inneren Oberfläche der Hülse 45 angeordnet, wobei diese Nut parallel zur Mittelachse verläuft. Die Nut 224 sollte zumindest so lang sein, wie die Amplitude der Pendelbewegung. Die Hülse bestimmt dadurch die Winkelposition des Verschlussschiebers 46 um die Mittelachse, lässt jedoch eine Bewegung des Verschlussschiebers 46 in Richtung der Mittelachse zu. Auf diese Weise kann der Verschlussschieber 46 durch Drehen der Hülse 45 zwischen der Geöffnet-Position und der Geschlossen-Position verschoben werden, ohne von der Hülse 45 an der Linearbewegung in Richtung der Mittelachse während des Bohrens gehindert zu werden.

Auch hier kann, wie in Figur 21 gezeigt, die äußere Hülse 45 einen hier nicht gezeigten Griff oder Knopf 44 aufweisen, mit dem sie drehbar ist.

Die Hülse 45 verdreht die Verschlussschieber 46 durch Formschluss, z. B. über Nut-Federverbindung zwischen Verschlusselement und Hülse 45, wobei die Nut 224 wie beschrieben in Richtung der Mittelachse, also in Bohrrichtung ausgerichtet ist. Die Verschlusselemente können wie beschrieben in der Hülse 45 auf und ab gleiten. Es sei darauf hingewiesen, dass die Verschlusselemente während des Bohrens mit den Bohrraspeln fest verbunden sind, die sich auf und ab bewegen. Der Verbindungsprozess erfolgt wieder dadurch, dass die Bohrraspeln 2 in die Lanzenaufnehmer 6 geschoben werden bis zum Anschlag und schließlich durch Drehung der Hülse Formschluss zwischen dem Lanzenaufnehmer, der Lanze 2 und dem Verschlusselement 46 erzeugt wird.

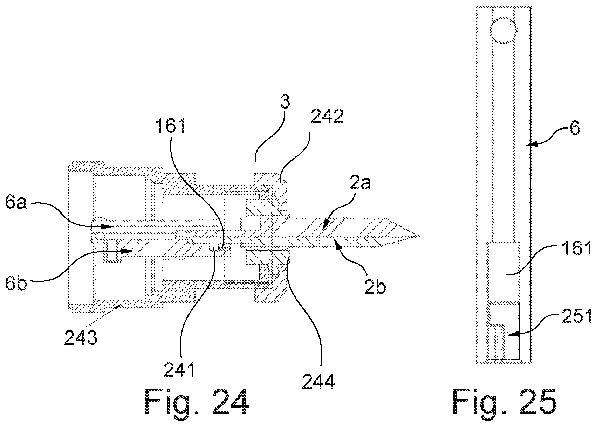

Die Figuren 24 und 25 zeigen eine weitere beispielhafte Ausgestaltung eines Schnellspannmechanismus 3, wie er im erfindungsgemäßen Bohradapter 1 eingesetzt werden kann.

Figur 25 zeigt dabei einen der Lanzenaufnehmer 6, hier die radial innenliegende Seite des Lanzenaufnehmers 6. Der Aufnahmekanal 161 weist hierbei in seiner Oberfläche eine Nut 251 auf, die beginnend an dem den Lanzen 2 zugewandten Ende des Lanzenaufnehmers 6 zunächst parallel zur Mittelachse verläuft und dann um 90° abknickt. Die Bohrlanzen 2a, 2b weisen entsprechend an ihren Einführelementen 47a einen Vorsprung 241 auf. Beim Einführen der Bohrlanze 2a, 2b in den entsprechenden Lanzenaufnehmer 6 wird dann der entsprechende Vorsprung 241 in die Nut 251 eingeschoben bis sie an dem den Lanzen 2 abgewandten Ende der Nut 251 anschlägt. Es wird dann die Bohrlanze 2a, 2b, 2c etwas gedreht, so dass der Vorsprung 241 im abgeknickten Bereich der Nut 251 sitzt. Hierdurch wird die Bohrlanze 2 im Lanzenaufnehmer 6 in Richtung der Mittelachse festgehalten. Bevorzugterweise sind die Nuten 251 in allen Lanzenaufnehmern 6 in die gleiche Richtung abgeknickt. Auf diese Weise ist es möglich, die Bohrlanzen 2a, 2b, 2c gemeinsam in die Lanzenaufnehmer 6a, 6b, 6c einzuführen und durch eine gemeinsame Drehung zu arretieren. Der hier beschriebene Mechanismus kann im Prinzip als Bajonettverschluss angesehen werden. Um die Lanzen 2a, 2b, 2c gegen Verdrehen zu sichern, ist im in Figur 24 gezeigten Beispiel eine Kappe 242 mit Innengewinde auf das Gehäuse 243 aufgeschraubt. Diese kann die vordere Linearführung 244 an das Gehäuse pressen und so eine Verdrehung der vorderen Linearführung 244 verhindern. Auf diese Weise wird eine Verdrehung der Lanzen 2a, 2b, 2c verhindert. Die Kappe 242 kann vor Bohrbeginn und nach dem Verdrehen der Lanzen 2a, 2b, 2c aufgeschraubt werden.

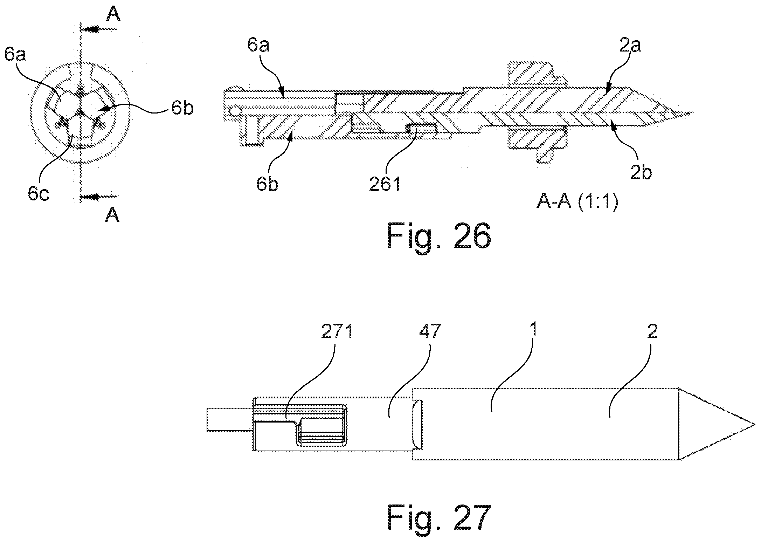

Die Figuren 26 und 27 zeigen eine ähnliche Ausgestaltung wie in den Figuren 24 und 25 gezeigt. Es ist hier jedoch eine Nut 271 in den Einführelementen 47 der Bohrraspeln 2 vorgesehen. Dabei verläuft die Nut 271 von einem der Spitze der Bohrlanze 2 abgewandten Stirnfläche zunächst parallel zur Mittelachse, um dann um 90° abzuknicken. Es können dann die Lanzenaufnehmer 6a, 6b jeweils eine positive Austragung 261 aufweisen, die in die Nut 271 hineinragt.

Beim Einführen der Lanzen 2a, 2b, 2c in die Lanzenaufnehmer gleitet die positive Austragung 261 in der Nut 271 der Bohrlanzen 2.

Im gezeigten Beispiel sind die Aufnahmekanäle 161 der Lanzenaufnehmer 6 so ausgestaltet, dass wenn die Lanzenaufnehmer 6a, 6b, 6c auf gleicher Höhe angeordnet sind, die Aufnahmekanäle 161 aller Lanzenaufnehmer 6a, 6b, 6c gemeinsam eine zylinderförmige Bohrung beschreiben. Entsprechend könne jene Bereiche der Einführelemente 47 der Bohrlanzen 2 als Zylindersegmente von 120° abzgl. eines Drehwinkels zur Arretierung (z.B. 15°) = (z.B.) 105° ausgestaltet sein. Dadurch kann die Lanze 2a, 2b, 2c vollständig eingeführt werden und dann verdreht werden, ohne dass das Zylindersegment des Einführelements das Zylindersegment der zylinderförmigen Bohrung (Aufnahmekanal) verlässt. Die Einführelemente 47 aller Bohrlanzen 2 zusammen beschreiben also teilweise einen Zylinder.

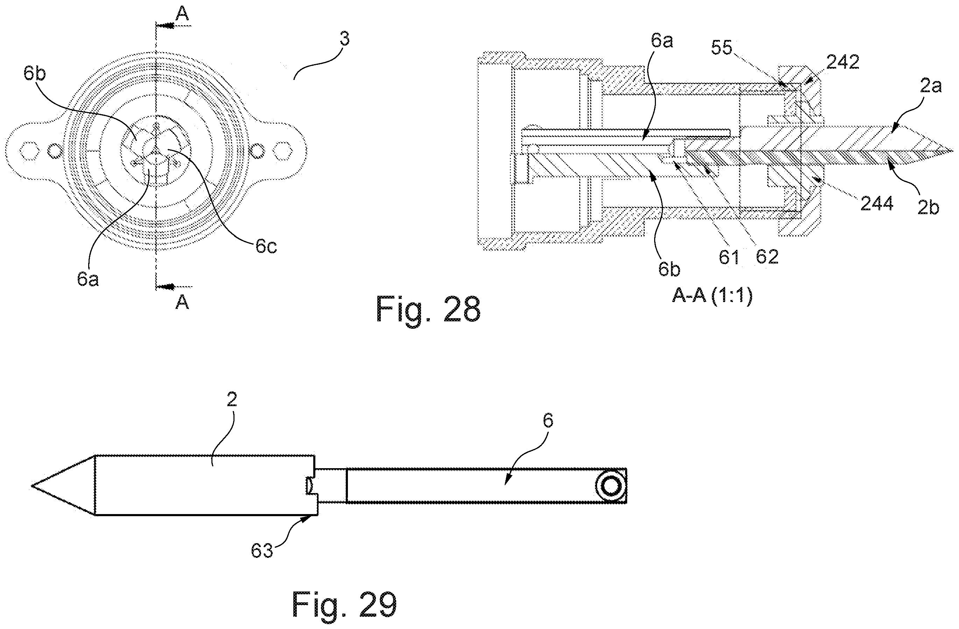

Die Figuren 28 und 29 beschreiben eine weitere mögliche Ausgestaltung eines Schnellspannmechanismus 3, wie er im erfindungsgemäßen Bohradapter 1 zum Einsatz kommen kann.

Hierbei können die Bohrlanzen 2 ein gemeinsames Außengewinde 62 aufweisen (gleiche Rotationsachse), welches in ein passendes Innengewinde in den Lanzenaufnehmern 6a, 6b, 6c geschraubt wird, solange bis ein Anschlag 63 erreicht wird. Der Anschlag 63 kann sich auf den Lanzenaufnehmern 6a, 6b, 6c befinden und so gesetzt sein, dass die Lanzen 2a, 2b, 2c in einer vordefinierten Position an dem Lanzenaufnehmer 6a, 6b, 6c gestoppt werden. Der Anschlag 63 verhindert dann eine weitere Verdrehung. Der Anschlag 63 ist so vorgesehen, dass alle Bohrraspeln 2a, 2b, 2c gleich weit im Gewinde eingeschraubt sind und gleichzeitig jedem Lanzenaufnehmer 6a, 6b, 6c eine Bohrlanze 2a, 2b, 2c zugeordnet ist, so dass die Bewegungen der einzelnen Bohrlanzen 2a, 2b, 2c beim Bohren nicht mit den Teilungsebenen zwischen den Lanzenaufnehmern 6a, 6b, 6c und Bohrlanzen 2a, 2b, 2c interferieren.

Zum Einschrauben ist zusätzlich eine Aussparung 61 in den Lanzenaufnehmern 6a, 6b, 6c vorgesehen, welche beim Eindrehen der Bohrlanzen 2a, 2b, 2c erlaubt, dass die Gewinde der Bohrlanzen 2a, 2b, 2c zyklisch die Gewinde der Lanzenaufnehmer 6a, 6b, 6c wechseln, ohne dabei mit den Bohrlanzenaufnehmern zu kollidieren. Dies ist vorteilhaft, da die unterschiedliche Position der Lanzenaufnehmer 6a, 6b, 6c zueinander sonst bei einer der Bohrlanzen 2a, 2b, 2c das Weiterdrehen verfrüht beenden würde. Bei den anderen Varianten des Schnellspannmechanismus 3 werden die Bewegungsfreiheitsgrade der Bohrlanzen 2a, 2b, 2c relativ zu den Lanzenaufnehmern 6a, 6b, 6c durch Formschluss der Bohrlanzen 2a, 2b, 2c und Bohrlanzenaufnehmer verhindert, bis auf den Bewegungsfreiheitsgrad in der Bohrrichtung. Hierfür sind die beschriebenen Verschlusselemente oder Nuten und/oder Austragungen zuständig. Im vorliegenden Beispiel verhindert die Gewindeverbindung auch zusätzlich die Bewegung entgegen der Bohrrichtung. Bei der hier beschriebenen Variante kann eine frontale Linearführung 244 frei drehbar sein (um die Bohr- bzw. Einschubachse), da mehrere Drehungen nötig sind. Auch hier wird diese durch das Anziehen einer Kappe 242 mit Gewinde 55 auf dem Gehäuse durch Kraftschluss gesichert.

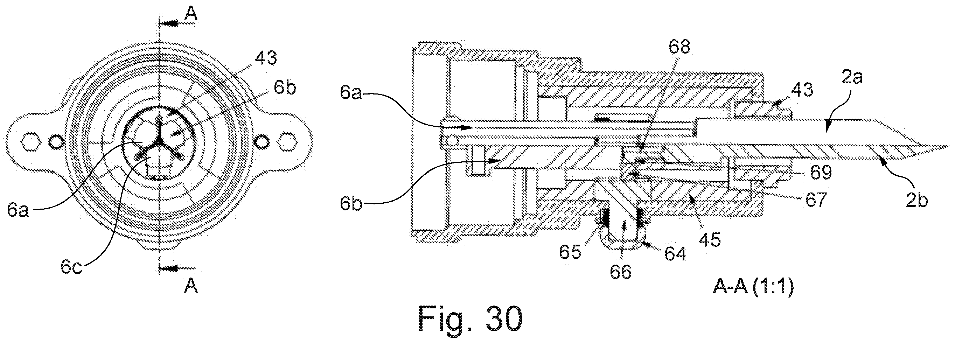

Figur 30 zeigt eine weitere mögliche Ausgestaltung eines Schnellspannmechanismus 3, der im erfindungsgemäßen Bohradapter 1 zur Anwendung kommen kann.

In diesem Beispiel wird der Formschluss in Bohrrichtung, durch den die Lanzen 2a, 2b in den Lanzenaufnehmern 6a, 6b gehalten werden durch einen Schnapphaken 68 an jeder der Bohrlanzen 2a, 2b erzeugt. Beim Einschieben der Bohrlanzen 2a, 2b wird dieser elastisch zur Mittelachse hin gebogen und rastet dann beim Anschlag angekommen ein. Figur 30 zeigt dabei den eingerasteten Zustand. Der Schnapphaken 68 weist hierfür eine senkrecht zur Mittelachse stehende Haltefläche auf, die auf einer Fläche 69 des Lanzenaufnehmers 6a, 6b aufliegt und so den Formschluss in Bohrrichtung, also in Richtung der Mittelachse, gewährleistet.

Für jede der Bohrlanzen 2a, 2b sind Elemente 66 vorgesehen, die radial im Gehäuse auf Höhe des Schnapphakens 68 der Bohrraspel 2a, 2b (im eingesetzten Zustand) eingebracht sind. Die Ausdehnung dieser Elemente in Richtung der Mittelachse ist so groß, dass sich der Schnapphaken immer auf Höhe dieser Elemente befindet, unabhängig von der Position der Lanzenaufnehmer 6a, 6b während der Pendelbewegung. Diese Elemente 66 berühren die Bohrraspeln 2a, 2b und die Lanzenaufnehmer 6a, 6b nicht und schränken sie nicht in der Bohrbewegung ein. Die Elemente 66 sind nach außerhalb des Gehäuses geführt und lassen sich in Richtung senkrecht zur Mittelachse verschieben.

Darüberhinaus sind gleitfähige Zusatzteile 67 vorgesehen, die in den Lanzenaufnehmern 6a, 6b auf Höhe des Schnapphakens 68 angebracht sind und in dessen Richtung, also im Wesentlichen radial, verschieblich sind. Um zusätzlich geführte Verschiebung zu gewährleisten ist hier zusätzlich eine für die Elemente 66 passende Aussparung in einer Hülse 45 vorgesehen. Das Element 66 kann dann beispielsweise durch einen Druckknopf 64 manuell aktuiert werden und durch eine Druckfeder 65 zurückgestellt werden, so dass die Elemente 66 bei der Bohrbewegung nicht mit den Bohrlanzen 2a, 2b oder den Lanzenaufnehmern 6a, 6b in Berührung sind. Andere Rückstellmöglichkeit wie beispielsweise Zugfedern oder ähnliches sind möglich.

Die frontale Linearführung 43 ist hier vorzugsweise verdrehsicher im Gehäuse gesichert, da diese nicht verdreht werden muss. Die Bohrraspeln 2a, 2b, 2c werden durch Einschieben in die Lanzenaufnehmer 6a, 6b bis zum Einrasten der Schnapphaken gesichert. Zum Lösen kann der entsprechende Druckknopf 64 gedrückt werden, wodurch der Formschluss der Schnapphaken 68 gelöst wird und die Bohrlanze 2 wieder herausgezogen werden kann.

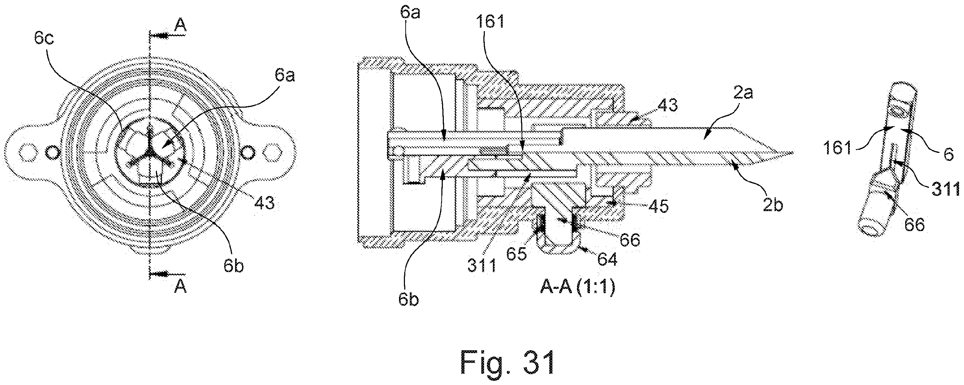

Figur 31 zeigt eine weitere beispielhafte Ausgestaltung eines Schnellspannmechanismus 3, der im erfindungsgemäßen Bohradapter 1 zum Einsatz kommen kann.

Im in Figur 31 gezeigten Beispiel weisen die Lanzenaufnehmer 6 eine Schlitzung auf, die sich durch die Wand des Aufnahmekanals 161 erstreckt. Dabei beginnt die Schlitzung 311 an jenem den Lanzen 2 zugewandtem Ende des entsprechenden Aufnahmekanals 161 und erstreckt sich über eine gewisse Strecke parallel zur Mittelachse in der Wand des Aufnahmekanals.

Es ist darüberhinaus ein Spreizelement 66 vorgesehen, das in gleicher radialer Richtung bezüglich der Mittelachse angeordnet ist wie die Schlitzung 311, und sich radial nach außen erstreckt. Das Spreizelement 66 hat dabei eine dem Schlitz 311 zugewandte Spitze.

In dieser Variante werden die Bohrlanzen 2 durch Kraftschluss in den Lanzenaufnehmern 6 gehalten. Das Spreizelement 66 kann beispielsweise über einen Druckknopf 64 von außen aktuiert werden, wobei eine unterhalb des Druccknopfes 64 angeordnete Feder 65 für die Rückstellung sorgt. Durch Drücken des Druckknopfes 64 wird die Spitze des Spreizelementes 66 in den Schlitz 311 gedrückt und dieser dadurch gespreizt. Dies vergrößert den Querschnitt des Aufnahmekanales 161, wodurch das Einführelement 47 der entsprechenden Bohrlanze 2 freigegeben wird und die Bohrlanze 2 entnommen werden kann. Die Spreizung des Spitzes 311 erfolgt dabei elastisch bzw. reversibel. Im gespreizten Zustand kann die Bohrlanze 2 leicht ein und wieder herausgeführt werden. Wird das Spreizelement 66 aus dem Schlitz 311 entfernt, übt der Lanzenaufnehmer 6 Druck auf das Einführelement der Bohrlanze 2 aus, so dass diese in radialer Richtung formschlüssig im Lanzenaufnehmer 6 sitzt und in Richtung der Mittelachse durch Kraftschluss gehalten wird.

## Patentansprüche

1. Bohradapter (1) zum Aufsetzen auf einen Antriebsmechanismus (A), wobei der Bohradapter (1) aufweist:
ein um eine Verbindungselementachse drehbares Verbindungselement (5), das mit dem Antriebsmechanismus (A) verbindbar ist, zumindest zwei um eine Mittelachse angeordnete Bohrlanzen (2a, 2b, 2c),
einen Linearisierungsmechanismus (4), mit dem eine Rotation des Verbindungselementes in oszillierende Bewegungen der zumindest zwei Bohrlanzen (2a, 2b, 2c) in Richtung parallel zu der Mittelachse übertragbar ist, und
einen Schnellspannmechanismus (3), der für jede der Bohrlanzen (2a, 2b, 2c) einen Lanzenaufnehmer (6a, 6b, 6c) aufweist,
wobei die Bohrlanzen (2a, 2b, 2c) jeweils an einem der Lanzenaufnehmer (6a, 6b, 6c) lösbar befestigbar sind.

2. Bohradapter nach dem vorhergehenden Anspruch,
wobei der Linearisierungsmechanismus ein mit dem Verbindungselement verbundenes oder gekoppeltes Führungssystem (11) aufweist, wobei der Linearisierungsmechanismus außerdem für jeden der Lanzenaufnehmer ein mit dem entsprechenden Lanzenaufnehmer gekoppeltes Abgriffselement (9a, 9b, 9c) aufweist, wobei die Abgriffselemente jeweils in eine Richtung parallel zur Mittelachse verschiebbar sind, wobei die Abgriffselemente mit dem Führungssystem gekoppelt sind, um bei Rotation des Verbindungselementes um die Verbindungselementachse in Richtung parallel zu der Mittelachse verschoben zu werden.

3. Bohradapter nach dem vorhergehenden Anspruch,
wobei der Linearisierungsmechanismus als Führungssystem zumindest eine Bahn oder Nut (8) aufweist, auf oder in welcher die Abgriffselemente um die Mittelachse herum gleiten oder abrollen, wobei vorzugsweise
die Bahn oder die Nut die Mittelachse umläuft und gegenüber einer Ebene, auf der die Mittelachse senkrecht steht, unterschiedlich geneigte Abschnitte aufweist, wobei vorzugsweise die Position der Bahn oder der Nut in Richtung der Mittelachse entlang ihres Umlaufes um die Mittelachse zumindest bereichsweise eine Sinusfunktion beschreibt.

4. Bohradapter nach einem der Ansprüche 2 oder 3,
wobei der Linearisierungsmechanismus als Führungssystem einen Ring (15) aufweist, der zumindest abschnittsweise gegenüber einer zur Mittelachse senkrechten Ebene geneigt ist und dessen äußerer Rand von den Abgriffselementen eingefasst wird.

5. Bohradapter nach einem der vorhergehenden Ansprüche,
wobei das Führungssystem einen die Mittelachse umlaufenden ersten Ring (15a) aufweist, der gegenüber der Mittelachse geneigt ist und fest gegenüber dem Verbindungselement ist,
wobei das Führungssystem außerdem einen zweiten Ring (15b) aufweist, der koaxial zum ersten Ring angeordnet ist und gegenüber dem ersten Ring beweglich gelagert ist,
wobei die Lanzenaufnehmer mit dem zweiten Ring jeweils über zumindest ein Gelenk (16a, 16b, 16c) verbunden sind, dessen Gelenkachse vorzugsweise senkrecht zur Mittelachse steht.

6. Bohradapter nach einem der vorhergehenden Ansprüche,
wobei das Verbindungselement an einem Ende des Verbindungselementes ein um die Verbindungselementachse verlaufendes Verbindungskegelrad (19) aufweist,
wobei der Linearisierungsmechanismus für jeden der Lanzenaufnehmer ein Abgriffskegelrad (20a) aufweist, dessen Drehachse senkrecht steht zur Verbindungselementachse und das mit dem Verbindungskegelrad in Eingriff ist,
wobei die Abgriffskegelräder jeweils mit einer um die Drehachse des entsprechenden Abgriffskegelrades drehbaren Kurvenscheibe (22a) verbunden sind,
wobei die Kurvenscheiben jeweils eine Nut (21a) in einer ihrer parallel zur Mittelachse orientierten Oberflächen aufweisen,
wobei jeweils an dem den Bohrlanzen abgewandten Ende der Lanzenaufnehmer eine Abgriffsrolle (23a) vorgesehen ist, die in der Nut der entsprechenden Kurvenscheibe liegt und deren Drehachse parallel liegt zur Drehachse des entsprechenden Abgriffskegelrads.

7. Bohradapter nach einem der Ansprüche 1 bis 3 oder 6,
wobei das Verbindungselement an einem Ende des Verbindungselementes ein um die Verbindungselementachse verlaufendes Verbindungskegelrad (19) aufweist,
wobei der Linearisierungsmechanismus für jeden der Lanzenaufnehmer ein Abgriffskegelrad (20a) aufweist, dessen Drehachse senkrecht steht zur Verbindungselementachse und das mit dem Verbindungskegelrad in Eingriff ist,
wobei die Abgriffskegelräder jeweils mit einem um die Drehachse des entsprechenden Abgriffskegelrades drehbaren Exzenter (28a) verbunden sind,
wobei die Exzenter jeweils an ihrem äußeren Rand in Richtung senkrecht zur Drehachse des entsprechenden Abgriffskegelrades mit einem Abgriffselement in Kontakt sind, das mit dem entsprechenden Lanzenaufnehmer verbunden ist.

8. Bohradapter nach einem der vorhergehenden Ansprüche,
wobei die Lanzenaufnehmer jeweils einen in Richtung parallel zur Mittelachse verlaufenden Aufnahmekanal (161a, 161b, 161c) aufweisen, in den ein Einführelement (47a) der entsprechenden Lanze eingeführt ist.

9. Bohradapter nach dem vorhergehenden Anspruch,
wobei die Aufnahmekanäle jeweils eine bezüglich der Mittelachse in radialer Richtung verlaufende erste Bohrung aufweisen,
wobei die Einführelemente jeweils eine in eingeführtem Zustand zur ersten Bohrung koaxiale zweite Bohrung (48a) aufweisen,
wobei der Schnellspannmechanismus außerdem an jedem der Lanzenaufnehmer ein Verschlusselement (46a, 46b, 46c) aufweist, dass in die jeweilige erste und die zweite Öffnung eingeschoben ist.

10. Bohradapter nach dem vorhergehenden Anspruch,
wobei der Schnellspannmechanismus außerdem eine äußere Hülse (45) aufweist, welche um die Lanzenaufnehmer herum angeordnet ist, wobei die Verschlusselemente jeweils Teil eines entsprechenden Verschlussschiebers (46a, 46b, 46c) sind, der zwischen der äußeren Hülse und dem entsprechenden Lanzenaufnehmer angeordnet ist,
wobei die äußere Hülse eine innere Oberfläche aufweist, die zumindest einen Geöffnet-Bereich mit einem ersten Abstand von der Mittelachse und zumindest einen Geschlossen-Bereich mit einem zweiten Abstand von der Mittelachse aufweist,
wobei der erste Abstand größer oder gleich der radialen Ausdehnung des entsprechenden Verschlussschiebers abzüglich der radialen Länge der entsprechenden ersten Bohrung ist und
wobei der zweite Abstand kleiner als die radiale Ausdehnung des entsprechenden Verschlussschiebers abzüglich der Länge der entsprechenden ersten Bohrung ist.

11. Bohradapter nach Anspruch 8,
wobei die Einführelemente der Lanzen jeweils einen Vorsprung aufweisen,
wobei die Aufnahmekanäle jeweils eine Nut in ihrer inneren Oberfläche aufweisen, die von einem den Lanzen zugewandten Rand des entsprechenden Aufnahmekanals zunächst parallel zu Mittelachse verläuft und dann in einer Kurve um einen Winkel von 90° oder mehr abknickt,
wobei jeweils der Vorsprung beim Einführen der entsprechenden Lanze in der Nut führbar ist.

12. Bohradapter nach Anspruch 8,
wobei die Aufnahmekanäle auf ihrer inneren Oberfläche jeweils einen Vorsprung aufweisen,
wobei die Einführelemente auf ihrer äußeren Oberfläche jeweils eine Nut aufweisen, die von einem einer Bearbeitungsspitze der entsprechenden Lanze abgewandten Rand des Einführelementes zunächst parallel zur Mittelachse verläuft und dann in einer Kurve um einen Winkel von 90° oder mehr abknickt,
wobei jeweils der Vorsprung beim Einführen der entsprechenden Lanze in der Nut führbar ist.

13. Bohradapter nach Anspruch 8,
wobei die Einführelemente jeweils an ihrem der Bohrlanze abgewandten Ende einen Schnapphaken (68) aufweisen,
wobei die Lanzenaufnehmer jeweils eine radiale Durchgangsöffnung auf jener Höhe in Richtung der Mittelachse aufweisen, wo der Schnapphaken in eingeführten Zustand des Einführelementes eine Haltefläche aufweist,
weiter in jeder der Durchgangsöffnungen aufweisend einen Haltestift, der in Richtung einer Längsachse der entsprechenden Durchgangsöffnung verschiebbar ist.

14. Bohradapter nach Anspruch 1,
wobei die Lanzenaufnehmer einen gemeinsamen Kanal mit einem Innengewinde koaxial zur Mittelachse einschließen,
wobei die Bohrlanzen jeweils ein Einführelement aufweisen,
wobei die Einführelemente aller Bohrlanzen ein gemeinsames Außengewinde (62) aufweisen, das durch die Einführelemente zusammengesetzt wird, wobei die Einführelemente mit dem gemeinsamen Außengewinde in das gemeinsame Innengewinde eingeschraubt sind.

15. Bohradapter nach Anspruch 8,
wobei die Lanzenaufnehmer jeweils einen Schlitz (311) radial nach außen aufweisen, der eine den entsprechenden Aufnahmekanal umschließende Wand durchstößt,
wobei die Einführelemente so bemessen sind, dass sie kraftschlüssig im entsprechenden Aufnahmekanal gehalten werden,
wobei der Schnellspannmechanismus für jeden der Lanzenaufnehmer ein keilförmiges Spreizelement (66) aufweist, dass in radialer Richtung verschiebbar gelagert ist und jeweils eine Kante aufweist, die in radialer Richtung über dem entsprechenden Schlitz angeordnet ist,
wobei das Spreizelement vorzugsweise durch eine Feder (65) in einer von dem Schlitz beabstandeten Position gehalten wird und gegen die Federkraft mit der Kante in den Schlitz verschiebbar ist.

16. Bohradapter nach einem der vorhergehenden Ansprüche, wobei die zumindest zwei Bohrlanzen aneinander mit parallelen Längsachsen anliegen und bei Anordnung auf gleicher Höhe zusammen eine Spitze mit einem kreisförmigen, rechteckigen, dreieckigen, sechseckigen, achteckigen oder sternförmigen Umfang bilden.

17. Bohrsystem aufweisend einen Bohradapter (1) nach einem der vorhergehenden Ansprüche,
weiter aufweisend eine mit dem Verbindungselement (5) verbundende Antriebsvorrichtung (A).

## Claims

1. A drill adapter (1) for placing on a drive mechanism (A), wherein the drill adapter (1) comprises:
a connecting element (5) rotatable about a connecting element axis, which connecting element can be connected to the drive mechanism (A),
at least two drilling rods (2a, 2b, 2c) arranged about a central axis,
a linearization mechanism (4), by which a rotation of the connecting element can be transferred into oscillating movements of the at least two drilling rods (2a, 2b, 2c) in a direction parallel to the central axis, and
a quick-clamping mechanism (3) which has a rod receiver (6a, 6b, 6c) for each of the drilling rods (2a, 2b, 2c),
wherein the drilling rods (2a, 2b, 2c) can each be attached in a detachable manner to one of the rod receivers (6a, 6b, 6c).

2. The drill adapter according to the preceding claim,
wherein the linearization mechanism comprises a guide system (11) connected or coupled to the connecting element, wherein the linearization mechanism also comprises, for each of the rod receivers, a tapping element (9a, 9b, 9c) coupled to the corresponding rod receiver,
wherein the tapping elements are each displaceable in a direction parallel to the central axis, wherein the tapping elements are coupled to the guide system, in order to be displaced on rotation of the connecting element about the connecting element axis in a direction parallel to the central axis.

3. The drill adapter according to the preceding claim,
wherein the linearization mechanism comprises at least one track or groove (8) as a guide system, on or in which the tapping elements slide or roll about the central axis, wherein preferably
the track or the groove runs about the central axis and has sections which are inclined differently with respect to a plane to which the central axis is perpendicular, wherein preferably the position of the track or the groove in the direction of the central axis describes a sine function at least in some sections along its circulation about the central axis.

4. The drill adapter according to any one of claims 2 or 3,
wherein the linearization mechanism comprises a ring (15) as a guide system, which ring is inclined at least partly relative to a plane perpendicular to a central axis and its outer edge is bordered by the tapping elements.

5. The drill adapter according to any one of the preceding claims,
wherein the guide system comprises a first ring (15a) circulating the central axis, which ring is inclined with respect to the central axis and is fixed relative to the connecting element,
wherein the guide system also comprises a second ring (15b) which is arranged to be coaxial to the first ring and is movably mounted relative to the first ring,
wherein the rod receivers are connected respectively to the second ring via at least one joint (16a, 16b, 16c), the joint axis of which is preferably perpendicular to the central axis.

6. The drill adapter according to any one of the preceding claims,
wherein the connecting element comprises at one end of the connecting element a connecting bevel gear (19) extending about said connecting element axis,
wherein the linearization mechanism comprises for each of the rod receivers a tapping bevel gear (20a), an axis of rotation of which is perpendicular to the connecting element axis and which is in engagement with the connecting bevel gear,
wherein the tapping bevel gears are each connected to a cam disc (22a) rotatable about the axis of rotation of the corresponding tapping bevel gear,
wherein the cam discs each have a groove (21a) in one of their surfaces oriented parallel to the central axis,
wherein a tapping roller (23a) is provided respectively at the end of the rod receivers facing away from the drilling rods, which tapping roller lies in the groove of the corresponding cam disc and the axis of rotation of which lies parallel to the axis of rotation of the corresponding tapping bevel gear.

7. The drill adapter according to any one of claims 1 to 3 or 6,
wherein the connecting element comprises at one end of the connecting element a connecting bevel gear (19) extending about said connecting element axis,
wherein the linearization mechanism comprises for each of the rod receivers a tapping bevel gear (20a), an axis of rotation of which is perpendicular to the connecting element axis and which is in engagement with the connecting bevel gear,
wherein the tapping bevel gears are connected respectively to an eccentric (28a) rotatable about the axis of rotation of the corresponding tapping bevel gear,
wherein the eccentrics are in contact respectively on their outer edge in a direction perpendicular to the axis of rotation of the corresponding tapping bevel gear with a tapping element which is connected to the corresponding rod receiver.

8. The drill adapter according to any one of the preceding claims,
wherein the rod receivers each comprise a receiving channel (161a, 161b, 161c) extending in a direction parallel to the central axis, into which a feed element (47a) of the corresponding rod is inserted.

9. The drill adapter according to the preceding claim,
wherein the receiving channels each comprise a first bore extending in radial direction with respect to the central axis,
wherein the feed elements each comprise a second bore (48a) coaxial to the first bore in an inserted state,
wherein the quick-clamping mechanism also comprises a locking element (46a, 46b, 46c) on each of the rod receivers, which is inserted into the respective first and second opening.

10. The drill adapter according to the preceding claim,
wherein the quick-clamping mechanism further comprises an outer sleeve (45) which is arranged around the rod receiver, wherein the locking elements are each part of a corresponding locking slide (46a, 46b, 46c), which is arranged between the outer sleeve and the corresponding rod receiver, wherein the outer sleeve has an inner surface which has at least one open-section at a first distance from the central axis and at least one close- section at a second distance from the central axis,
wherein the first distance is greater than or equal to the radial extension of the corresponding locking slide minus the radial length of the corresponding first bore and
wherein the second distance is smaller than the radial extension of the corresponding locking slide minus the length of the corresponding first bore.

11. The drill adapter according to claim 8,
wherein the feed elements of the rods each comprise a projection,
wherein the receiving channels each have a groove in their inner surface, which groove initially runs parallel to the central axis from an edge of the corresponding receiving channel facing the rods and then bends in a curve through an angle of 90° or more,
wherein in each case the projection can be guided in the groove when inserting the corresponding rod.

12. The drill adapter according to claim 8,
wherein the receiving channels each have a projection on their inner surface,
wherein the feed elements each have a groove on their outer surface which groove initially runs parallel to the central axis from an edge of the feed element facing away from a machining tip of the corresponding rod and then bends in a curve through an angle of 90° or more,
wherein in each case the projection can be guided in the groove when inserting the corresponding rod.

13. The drill adapter according to claim 8,
wherein the feed elements each comprise a snap hook (68) at their end facing away from the drilling rod,
wherein the rod receivers each have a radial through opening at that height in the direction of the central axis, where the snap hook has a retaining surface in the inserted state of the feed element,
further comprising a retaining pin in each of the through openings which is displaceable in the direction of a longitudinal axis of the corresponding through opening.

14. The drill adapter according to claim 1,
wherein the rod receivers have a common channel with an internal thread coaxial to the central axis,
wherein the drilling rods each have a feed element,
wherein the feed elements of all drilling rods have a common external thread (62), which is assembled by the feed elements, wherein the feed elements with the common external thread are screwed into the common internal thread.

15. The drill adapter according to claim 8,
wherein the rod receivers each have a slot (311) radially outwards which pierces a wall enclosing the corresponding receiving channel,
wherein the feed elements are dimensioned to be held by force-fitting in the corresponding receiving channel,
wherein the quick-clamping mechanism for each of the rod receivers comprises a wedge-shaped expansion element (66) which is mounted displaceably in radial direction and has an edge respectively which is arranged in radial direction above the corresponding slot, wherein the expansion element is preferably held by a spring (65) in a position spaced from the slot and is displaceable into the slot against the spring force with the edge.

16. The drill adapter according to any one of the preceding claims,
wherein the at least two drilling rods abut on one another with parallel longitudinal axes and when arranged at the same level together form a tip with a circular, rectangular, triangular, hexagonal, octagonal or star-shaped circumference.

17. A drilling system comprising a drill adapter (1) according to any one of the preceding claims,
further comprising a drive device (A) connecting to the connecting element (5).

## Revendications

1. Adaptateur de perçage (1) destiné à être monté sur un mécanisme d'entraînement (A), dans lequel l'adaptateur de perçage (1) présente :
un élément de liaison (5) pouvant tourner autour d'un axe d'élément de liaison, qui peut être relié au mécanisme d'entraînement (A),
au moins deux lances de perçage (2a, 2b, 2c) disposées autour d'un axe central,
un mécanisme de linéarisation (4) avec lequel une rotation de l'élément de liaison peut être transmise en mouvements oscillants des au moins deux lances de perçage (2a, 2b, 2c) dans la direction parallèle à l'axe central, et
un mécanisme de serrage rapide (3) qui présente un récepteur de lance (6a, 6b, 6c) pour chacune des lances de perçage (2a, 2b, 2c),
dans lequel les lances de perçage (2a, 2b, 2c) peuvent être fixées chacune de manière amovible à l'un des récepteurs de lance (6a, 6b, 6c).

2. Adaptateur de perçage selon la revendication précédente,
dans lequel le mécanisme de linéarisation présente un système de guidage (11) relié ou couplé à l'élément de liaison, dans lequel le mécanisme de linéarisation présente en outre, pour chacun des récepteurs de lance un élément de prise (9a, 9b, 9c) couplé au récepteur de lance correspondant,
dans lequel les éléments de prise sont chacun déplaçables dans une direction parallèle à l'axe central, dans lequel les éléments de prise sont couplés au système de guidage pour être déplacés dans une direction parallèle à l'axe central lors de la rotation de l'élément de liaison autour de l'axe de l'élément de liaison.

3. Adaptateur de perçage selon la revendication précédente,
dans lequel le mécanisme de linéarisation présente, en tant que système de guidage, au moins une piste ou rainure (8) sur ou dans laquelle les éléments de prise glissent ou roulent autour de l'axe central, dans lequel de préférence
la bande ou la rainure tourne autour de l'axe central et présente des sections inclinées différemment par rapport à un plan sur lequel l'axe central est perpendiculaire, la position de la bande ou de la rainure dans la direction de l'axe central le long de sa rotation autour de l'axe central décrit de préférence au moins par zones une fonction sinusoïdale.

4. Adaptateur de perçage selon l'une quelconque des revendications 2 ou 3,
dans lequel le mécanisme de linéarisation présente comme système de guidage un anneau (15) qui est incliné au moins par sections par rapport à un plan perpendiculaire à l'axe central et dont le bord extérieur est bordé par les éléments de prise.

5. Adaptateur de perçage selon l'une quelconque des revendications précédentes,
dans lequel le système de guidage présente un premier anneau (15a) entourant l'axe central, qui est incliné par rapport à l'axe central et est fixe par rapport à l'élément de liaison,
dans lequel le système de guidage présente en outre un deuxième anneau (15b) qui est disposé coaxialement au premier anneau et est monté mobile par rapport au premier anneau,
dans lequel les récepteurs de lance sont reliés au deuxième anneau respectivement par l'intermédiaire d'au moins une articulation (16a, 16b, 16c) dont l'axe d'articulation est de préférence perpendiculaire à l'axe central.

6. Adaptateur de perçage selon l'une quelconque des revendications précédentes,
dans lequel l'élément de liaison présente à une extrémité de l'élément de liaison une roue conique de liaison (19) s'étendant autour de l'axe de l'élément de liaison,
dans lequel le mécanisme de linéarisation présente, pour chacun des récepteurs de lance, une roue conique de prise (20a) dont l'axe de rotation est perpendiculaire à l'axe de l'élément de liaison et qui est en prise avec la roue conique de liaison,
dans lequel les roues coniques de prise sont chacune reliées à une came (22a) pouvant tourner autour de l'axe de rotation de la roue conique de prise correspondante,
dans lequel les cames présentent chacune une rainure (21a) dans l'une de leurs surfaces orientées parallèlement à l'axe central,
dans lequel respectivement un galet de prise (23a) est prévu à l'extrémité des récepteurs de lance opposée aux lances de perçage, qui se trouve dans la rainure de la came correspondante et dont l'axe de rotation est parallèle à l'axe de rotation de la roue conique de prise correspondante.

7. Adaptateur de perçage selon l'une quelconque des revendications 1 à 3 ou 6,
dans lequel l'élément de liaison présente à une extrémité de l'élément de liaison une roue conique de liaison (19) s'étendant autour de l'axe de l'élément de liaison,
dans lequel le mécanisme de linéarisation présente, pour chacun des récepteurs de lance, une roue conique de prise (20a) dont l'axe de rotation est perpendiculaire à l'axe de l'élément de liaison et qui est en prise avec la roue conique de liaison,
dans lequel les roues coniques de prise sont chacune reliées à un excentrique (28a) pouvant tourner autour de l'axe de rotation de la roue conique de prise correspondante,
dans lequel les excentriques sont chacun en contact, au niveau de leur bord extérieur, dans une direction perpendiculaire à l'axe de rotation de la roue conique de prise correspondante, avec un élément de prise qui est relié au récepteur de lance correspondant.

8. Adaptateur de perçage selon l'une quelconque des revendications précédentes,
dans lequel les récepteurs de lance présentent chacun un canal de réception (161a, 161b, 161c) s'étendant dans la direction parallèle à l'axe central, dans lequel est introduit un élément d'insertion (47a) de la lance correspondante.

9. Adaptateur de perçage selon la revendication précédente,
dans lequel les canaux de réception présentent chacun un premier alésage s'étendant dans la direction radiale par rapport à l'axe central,
dans lequel les éléments d'insertion présentent chacun un deuxième alésage (48a) coaxial au premier alésage à l'état inséré,
dans lequel le mécanisme de serrage rapide présente en outre, sur chacun des récepteurs de lance, un élément de fermeture (46a, 46b, 46c) qui est inséré dans la première et la deuxième ouverture respective.

10. Adaptateur de perçage selon la revendication précédente,
dans lequel le mécanisme de serrage rapide présente en outre un manchon extérieur (45), lequel est disposé autour des récepteurs de lance, dans lequel les éléments de fermeture font chacun partie d'un coulisseau de fermeture (46a, 46b, 46c) correspondant qui est disposé entre le manchon extérieur et le récepteur de lance correspondant, dans lequel le manchon extérieur a une surface intérieure, qui présente au moins une zone ouverte à une première distance de l'axe central et au moins une zone fermée à une deuxième distance de l'axe central,
dans lequel la première distance est supérieure ou égale à l'étendue radiale du coulisseau de fermeture correspondant moins la longueur radiale du premier trou correspondant, et
dans lequel la deuxième distance est inférieure à l'étendue radiale du coulisseau de fermeture correspondant moins la longueur du premier trou correspondant.

11. Adaptateur de perçage selon la revendication 8,
dans lequel les éléments d'insertion des lances présentent chacun une saillie,
dans lequel les canaux de réception présentent chacun une rainure dans leur surface intérieure qui, à partir d'un bord du canal de réception correspondant tourné vers les lances, s'étend d'abord parallèlement à l'axe central et se plie ensuite en une courbe d'un angle de 90° ou plus,
dans lequel respectivement la saillie peut à chaque fois être guidée dans la rainure lors de l'introduction de la lance correspondante.

12. Adaptateur de perçage selon la revendication 8,
dans lequel les canaux de réception présentent chacun une saillie sur leur surface intérieure,
dans lequel les éléments d'insertion présentent chacun sur leur surface extérieure une rainure qui, à partir d'un bord de l'élément d'insertion opposé à une pointe d'usinage de la lance correspondante, s'étend d'abord parallèlement à l'axe central, puis se plie en une courbe d'un angle de 90° ou plus,
dans lequel respectivement la saillie peut à chaque fois être guidée dans la rainure lors de l'introduction de la lance correspondante.

13. Adaptateur de perçage selon la revendication 8,
dans lequel les éléments d'insertion présentent chacun un crochet d'encliquetage (68) à leur extrémité opposée à la lance de perçage,
dans lequel les récepteurs de lance présentent chacun une ouverture de passage radiale à la hauteur, dans la direction de l'axe central, où le crochet d'encliquetage présente une surface de retenue à l'état inséré de l'élément d'insertion,
présentant en outre, dans chacune des ouvertures de passage, une broche de retenue qui peut se déplacer dans la direction d'un axe longitudinal de l'ouverture de passage correspondante.

14. Adaptateur de perçage selon la revendication 1,
dans lequel les récepteurs de lance incluent un canal commun avec un filetage intérieur coaxial à l'axe central,
dans lequel les lances de perçage présentent chacune un élément d'insertion,
dans lequel les éléments d'insertion de toutes les lances de perçage présentent un filetage extérieur (62) commun qui est assemblé par les éléments d'insertion, dans lequel les éléments d'insertion avec le filetage extérieur commun sont vissés dans le filetage intérieur commun.

15. Adaptateur de perçage selon la revendication 8,
dans lequel les récepteurs de lance présentent chacun une fente (311) radialement vers l'extérieur, qui perce une paroi entourant le canal de réception correspondant,
dans lequel les éléments d'insertion sont dimensionnés de manière à être maintenus par adhérence dans le canal de réception correspondant,
dans lequel le mécanisme de serrage rapide présente, pour chacun des récepteurs de lance, un élément d'écartement en forme de coin (66) qui est supporté de manière à pouvoir se déplacer dans la direction radiale et présente respectivement un bord qui est disposé dans la direction radiale au-dessus de la fente correspondante, dans lequel l'élément d'écartement est de préférence maintenu par un ressort (65) dans une position espacée de la fente et peut être déplacé dans la fente avec le bord à l'encontre de la force du ressort.

16. Adaptateur de perçage selon l'une quelconque des revendications précédentes, dans lequel les au moins deux lances de perçage sont adjacentes l'une à l'autre avec des axes longitudinaux parallèles et, lorsqu'elles sont disposées à la même hauteur, forment ensemble une pointe avec un périmètre circulaire, rectangulaire, triangulaire, hexagonal, octogonal ou en forme d'étoile.

17. Système de perçage présentant un adaptateur de perçage (1) selon l'une quelconque des revendications précédentes,
présentant en outre un dispositif d'entraînement (A) relié à l'élément de liaison (5).
